# EUROPEAN PATENT SPECIFICATION

(11) **EP 4 300 610 B1**
(45) Date of publication and mention of the grant of the patent: **11.11.2026**
(21) Application number: 22759239.1
(22) Date of filing: 28.01.2022
(51) Int. Cl.: C07D 471/04, H05B 33/12, H10K 50/16, H10K 85/60

(54) **ORGANIC EL ELEMENT MATERIAL, ORGANIC EL ELEMENT, DISPLAY DEVICE, AND ILLUMINATION DEVICE**
ORGANISCHES EL-ELEMENTMATERIAL, ORGANISCHES EL-ELEMENT, ANZEIGEVORRICHTUNG UND BELEUCHTUNGSVORRICHTUNG
MATÉRIAU D'ÉLÉMENT ÉLECTROLUMINESCENT ORGANIQUE, ÉLÉMENT ÉLECTROLUMINESCENT ORGANIQUE, DISPOSITIF D'AFFICHAGE ET DISPOSITIF D'ÉCLAIRAGE

(30) Priority: 24.02.2021 JP 2021027160
(43) Date of publication of application: 03.01.2024
(73) Proprietor: Toray Industries, Inc., Tokyo 103-8666 (JP)
(72) Inventor: TOKUDA, Takashi, Otsu-shi, Shiga 520-8558 (JP); OKANO, Tsubasa, Kamakura-shi, Kanagawa 248-8555 (JP); NAGAO, Kazumasa, Otsu-shi, Shiga 520-8558 (JP)
(74) Representative: Hoefer & Partner Patentanwälte mbB
(86) International application number: PCT/JP2022/003268
(87) International publication number: WO 2022/181197

(56) References cited:
- EP-A1- 2 983 227
- WO-A1-2016/121597
- WO-A1-2019/093547
- WO-A1-2020/218648
- JP-A- 2019 096 823
- JP-A- 2019 161 218
- JP-A- 2020 508 580
- JP-A- 2020 509 586
- KR-A- 20190 053 354
- KR-A- 20190 053 562
- KR-B1- 102 020 842
- BARANOFF ETIENNE ET AL: "A pseudo-rotaxane based on an iridium (III)-copper(I) dyad", NEW JOURNAL OF CHEMISTRY,20080101ROYAL SOCIETY OF CHEMISTRY, GB, vol. 28, no. 9, 1 January 2004 (2004-01-01), pages 1091 - 1095, XP093228966, ISSN: 1144-0546, DOI: 10.1039/B407291J

## Description

### [Technical Field]

The present invention relates to an organic EL element material having a specific structure, and an organic EL element, a display device and an illumination device using the same.

### [Background Art]

In recent years, organic EL elements have been steadily put into practical use, such as being adopted for displays of televisions and smartphones. However, existing organic EL elements still have many technical problems. Of these, achieving both highly efficient light emission and extending the life of the organic EL element is a major issue.

There have hitherto been developed, as compounds to solve these problems, phenanthroline derivatives which have a terpyridine skeleton and substituted with a specific aryl group (see, for example, Patent Literature 1), phenanthroline derivatives having a pyrene skeleton (see, for example, Patent Literature 2), phenanthroline derivatives having a dibenzofuran skeleton (see, for example, Patent Literatures 3 and 4), phenanthroline derivatives having a specific arylene group and heteroaryl group (see, for example, Patent Literature 5) and the like.

### [Citation List]

### [Patent Literature]

[Patent Literature 1]
   WO 2016/121597 A
[Patent Literature 2]
   Korean Patent Publication No. 10-2020-0064423
[Patent Literature 3]
   WO 2020-506892 W
[Patent Literature 4]
   WO 2020/218648 A
[Patent Literature 5]
   EP 2983227 A

### [Summary of Invention]

### [Technical Problem]

According to Patent Literatures 1 to 5, it is possible to obtain an organic EL element, which enables high luminance efficiency, low driving voltage and is excellent in durability, by phenanthroline derivatives linked with a terpyridine skeleton, a pyrene skeleton, a dibenzofuran skeleton, or a specific aryl group, arylene group or heteroaryl group. However, the luminance efficiency and durability required for the organic EL element have been increasing in recent years, and there is a growing need for technologies that can achieve both higher luminance efficiency and longer durability life.

In light of these problems of the prior art, an object of the present invention is to provide an organic EL element excellent in luminance efficiency and durability life.

### [Solution to Problem]

The present invention, which is defined in the appended claims, is directed to an organic EL element material represented by the following general formula (1).

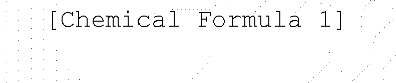

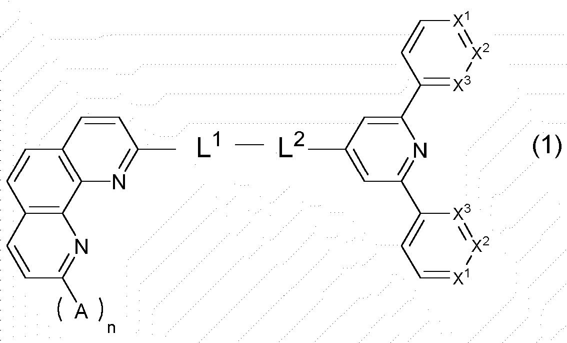

In the general formula (1), any one of X³ is a nitrogen atom, and X¹ and X² are methine groups. L¹ is a substituted or unsubstituted phenylene group, a substituted or unsubstituted naphthylene group, or a substituted or unsubstituted anthrylene group, L² is a single bond, a substituted or unsubstituted phenylene group, a substituted or unsubstituted naphthylene group, or a substituted or unsubstituted anthrylene group. However, when these groups are substituted, the substituent is an alkyl group or an alkoxy group. A is a phenyl group or a pyridyl group, and n is 1.

### [Advantageous Effects of Invention]

According to the present invention, it is possible to provide an organic EL element excellent in luminance efficiency and durability life.

### [Description of Embodiments]

Hereinafter, preferred embodiments of an organic EL element material, an organic EL element, a display device and an illumination device according to the present invention will be described in detail.

### (Organic EL Element Material represented by General Formula (1))

The organic EL element material represented by the general formula (1), which is one embodiment of the present invention, comprises a phenanthroline derivative shown below, and represents a material used for any of the layers constituting the organic EL element. In other words, the organic EL element material means the application of the phenanthroline derivative represented by the following general formula (1).

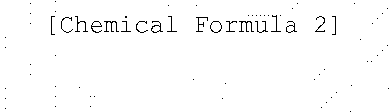

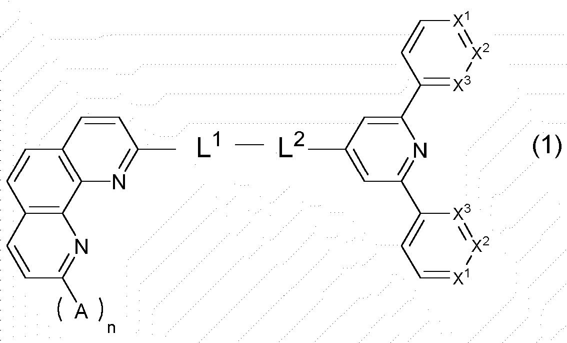

In the general formula (1), X³ is a nitrogen atom, and X¹ and X² are methine groups. L¹ is a substituted or unsubstituted phenylene group, a substituted or unsubstituted naphthylene group, or a substituted or unsubstituted anthrylene group, L² is a single bond, a substituted or unsubstituted phenylene group, a substituted or unsubstituted naphthylene group, or a substituted or unsubstituted anthrylene group. However, when these groups are substituted, the substituent is an alkyl group or an alkoxy group. A is a phenyl group or a pyridyl group, and n is 1.

"Unsubstituted" in the case of "substituted or unsubstituted" means that hydrogen atoms are linked, and "substituted" means that at least a portion of the hydrogen atoms are substituted. The hydrogen atom may be a deuterium atom. The same applies to "substituted or unsubstituted" in the compounds or partial structures thereof described below.

The alkyl group represents, for example, a saturated aliphatic hydrocarbon group such as a methyl group, an ethyl group, an n-propyl group, an isopropyl group, an n-butyl group, a sec-butyl group or a tert-butyl group, which may or may not have a substituent. The number of carbon atoms of the alkyl group is not particularly limited, but is usually in a range of 1 or more and 20 or less, and more preferably 1 or more and 8 or less, in view of the ease of availability and cost.

The alkoxy group represents, for example, a group in which an alkyl group is linked to oxygen, such as a methoxy group, an ethoxy group, an n-propoxy group, an isopropoxy group, an n-butoxy group, a sec-butoxy group or a tert-butoxy group, which may or may not have a substituent. The number of carbon atoms of the alkoxy group is not particularly limited, but is usually in a range of 1 or more and 20 or less, and more preferably 1 or more and 8 or less, in view of the ease of availability and cost.

Patent Literatures 1 to 5 disclose, as a conventional compound containing a nitrogen-containing aromatic heterocyclic ring and a polycyclic aromatic hydrocarbon, for example, compounds V, W, X, Y and Z represented by the following formulas.

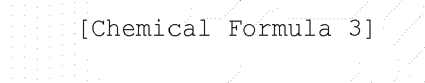

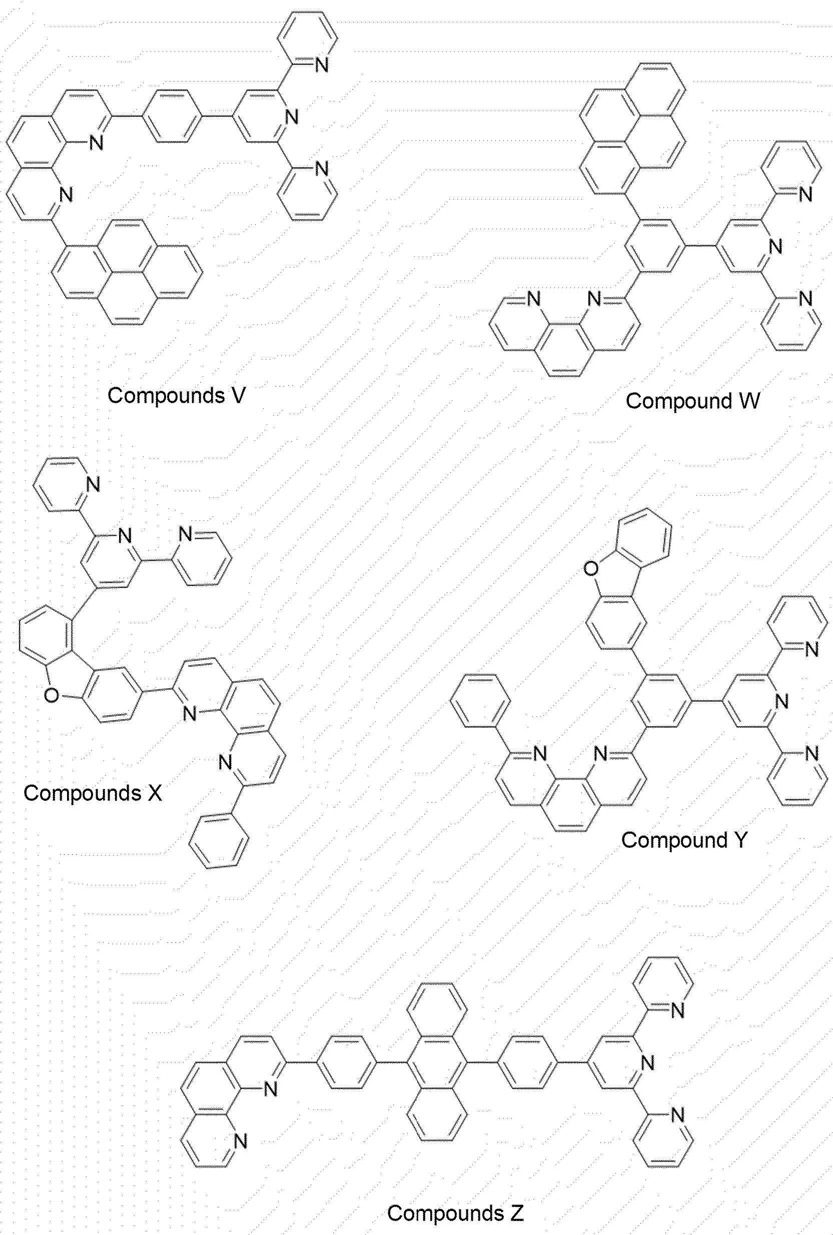

However, even an element in which these compounds are used as an organic EL element material for an electron injection layer, an electron transporting layer or a charge generation layer has not yet achieved sufficient performance to meet properties required in recent years, and there is a need to create compounds which enables further improvement in performance in view of the luminance efficiency and durability life.

For example, since compounds in which a phenanthrolinyl group is substituted with a pyrenyl group, such as a compound V, have a bulky substituent in the vicinity of a nitrogen atom on a highly coordinating phenanthrolinyl group, the compounds tend to cause deterioration of the coordinating property of a phenanthrolinyl group to the metal atom. Therefore, when the compound V is used together with the metal atom, the film stability deteriorates and the driving voltage increases, leading to problems such as deterioration of the luminance efficiency and durability life. Since compounds having a bulky aromatic substituent on a linking group between a terpyridyl group and a phenanthrolinyl group, such as compounds W and Y, are less effective in improving molecular interaction due to bulkiness thereof, the driving voltage increases, leading to problems with the luminance efficiency and durability life. Since compounds in which a terpyridyl group and a phenanthrolinyl group are linked by a dibenzofuranylene group, such as the compound X, have too high crystallinity due to high planarity of the phenanthrolinyl group and a linking group, the driving voltage increases, leading to problems with the luminance efficiency and durability life. Since compounds in which a terpyridyl group and a phenanthrolinyl group are linked by three arylene groups, such as the compound Z, also have too high crystallinity due to high planarity thereof, the driving voltage increases, leading to problems with the luminance efficiency and durability life.

The present inventors focused their attention on the effects of a phenanthrolinyl group, a terpyridyl group and a linking group thereof in the study of improving the organic EL element material. Both the phenanthrolinyl group and the terpyridyl group have high electron transportability and are highly coordinating substituents to the metal atom.

The organic EL element material represented by the general formula (1) is capable of selecting a phenylene group, a naphthylene group or an anthrylene group as L¹ and selecting a single bond, a phenylene group, a naphthylene group or an anthrylene group as L², thus making it easier to conjugate the phenanthrolinyl group, the terpyridyl group and the linking group thereof, leading to an enhancement in charge transportability of the compound as a whole. Therefore, when used for the organic EL element, the driving voltage can be reduced and the luminance efficiency can be improved. Since it is possible to enhance the coordinating property to the metal atom, when the organic EL element material represented by the general formula (1) is used for a metal-doped layer in the organic EL element, a stable layer can be formed. As used herein, the metal-doped layer refers to a layer obtained by doping any of the layers constituting the organic EL element with metal. In particular, when the organic EL element material represented by the general formula (1) is used for an electron transporting layer, an electron injection layer or a charge generation layer, these layers exhibit more stable and excellent performance.

In the general formula (1), L¹ is a substituted or unsubstituted phenylene group, a substituted or unsubstituted naphthylene group, or a substituted or unsubstituted anthrylene group, and L² is a single bond, a substituted or unsubstituted phenylene group, a substituted or unsubstituted naphthylene group, or a substituted or unsubstituted anthrylene group. When L¹ is substituted, the substituent is an alkyl group or an alkoxy group. These substituents are preferable since they can improve the stability of the compound without deteriorating the charge transportability of the compound. From the viewpoint of enhancing the film quality stability and further improving the luminance efficiency and durability life, L¹ or L² is preferably a naphthylene group. When L² is a single bond, it is possible to enhance the interaction between the phenanthrolinyl group and the terpyridyl group, leading to more improvement in luminance efficiency and durability life.

In the general formula (1), X³ is a nitrogen atom, and X¹ and X² are methine groups. From the viewpoint of enhancing the coordinating property to the metal atom and forming a more stable layer, X³ is a nitrogen atom. By forming a more stable layer, it is possible to drive at low voltage and to further improve the durability life.

In the general formula (1), n is 1, A is a phenyl group or a pyridyl group. Since n is 1, the stability of the compound can be improved because of being substituted at the highly reactive 2- and 9-positions of the substitution positions of phenanthroline. By selecting a phenyl group or a pyridyl group as A, when used for the metal-doped layer, it is possible to form a more stable layer with higher metal coordinating property due to high coordinating property of the nitrogen atom on the phenanthrolinyl group. Therefore, the driving voltage can be further reduced and the durability life can be improved. From the viewpoint of enhancing the stability and durability life of the element, A is preferably a phenyl group.

The molecular weight of the organic EL element material represented by the general formula (1) is preferably 400 or more from the viewpoint of suppressing crystallization and improving the stability of film quality. Meanwhile, the molecular weight of the organic EL element material represented by the general formula (1) is preferably 640 or less from the viewpoint of improving the processability during sublimation purification and vapor deposition.

Examples of the organic EL element material represented by the general formula (1) include compounds composed of the compounds shown below. The following are illustrative examples, and compounds other than those specified herein can also be preferably used as long as they are represented by the general formula (1).

### [Chemical Formula 4]

The compounds of Chemical Formula 4 do not fall under the claimed scope.

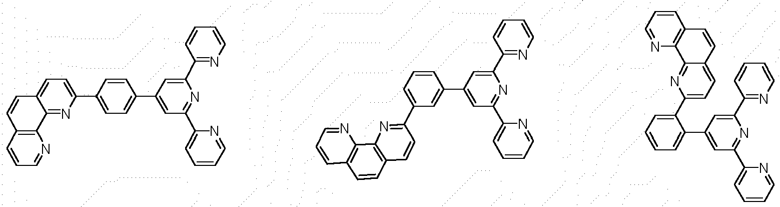

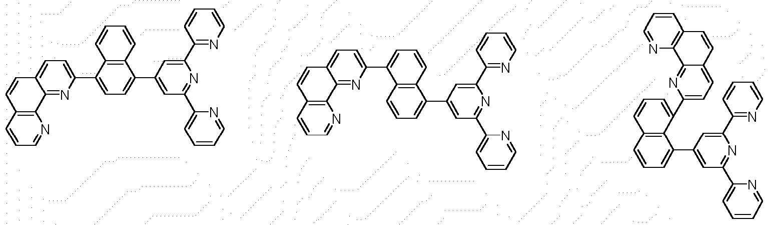

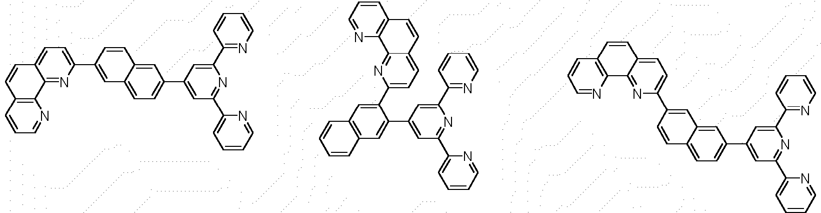

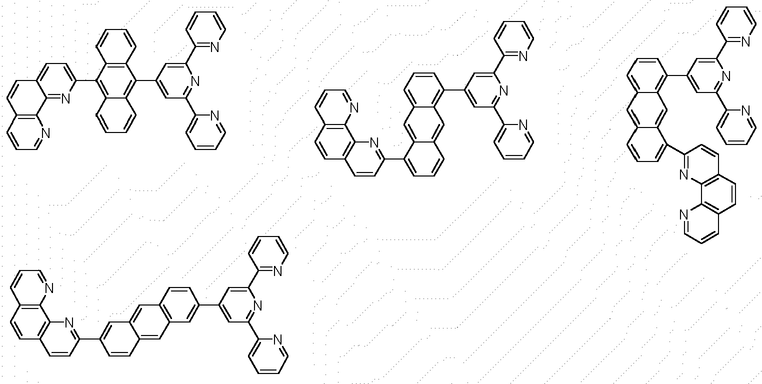

### [Chemical Formula 5]

The compounds of Chemical Formula 5 do not fall under the claimed scope.

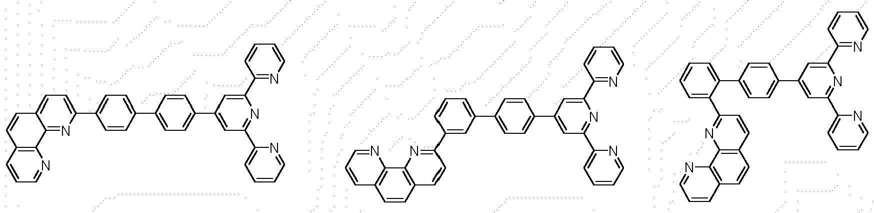

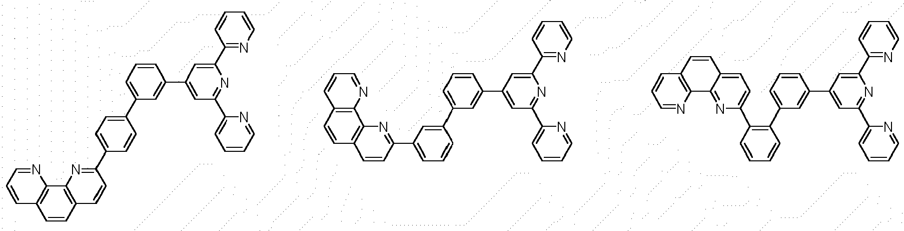

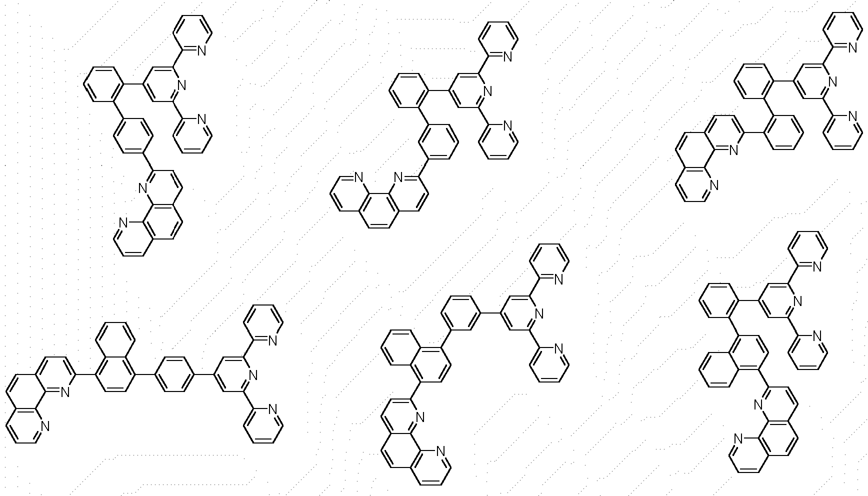

### [Chemical Formula 6]

The compounds of Chemical Formula 6 do not fall under the claimed scope.

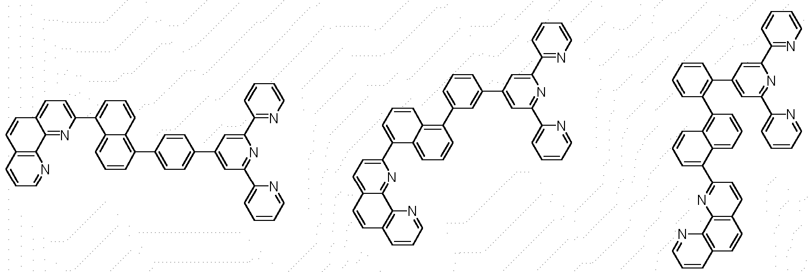

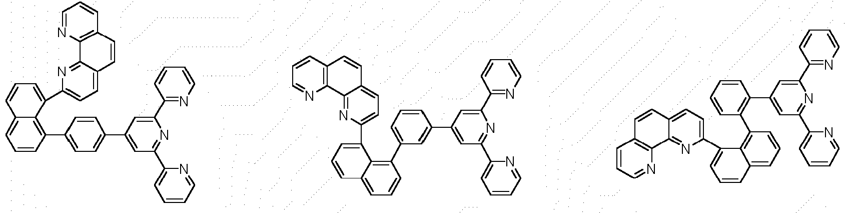

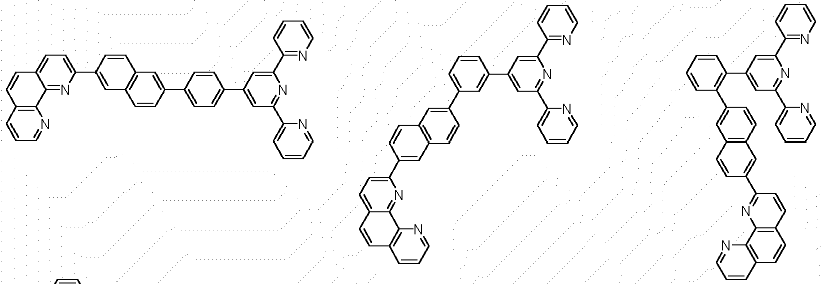

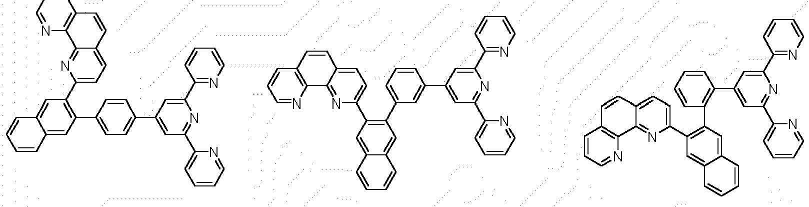

### [Chemical Formula 7]

The compounds of Chemical Formula 7 do not fall under the claimed scope.

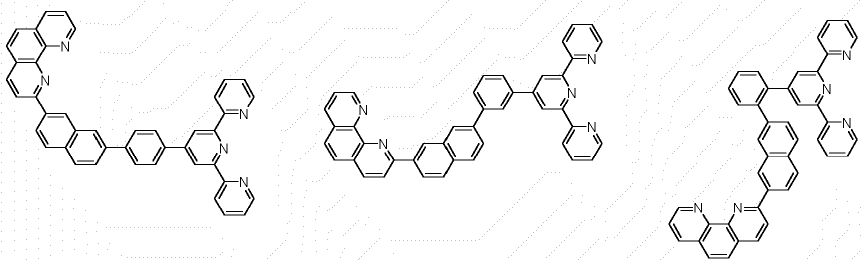

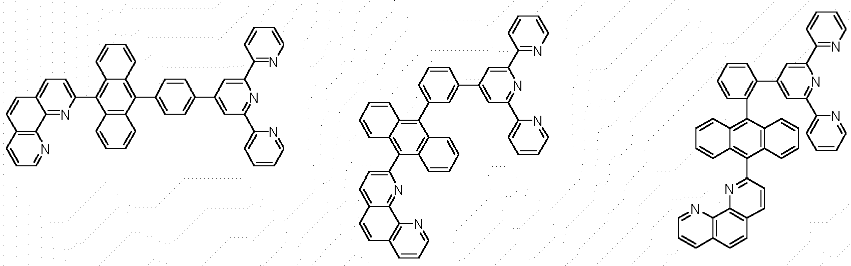

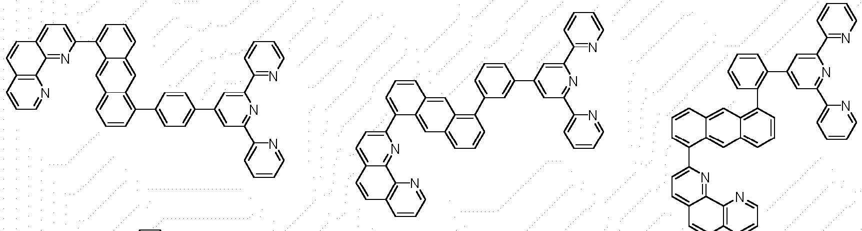

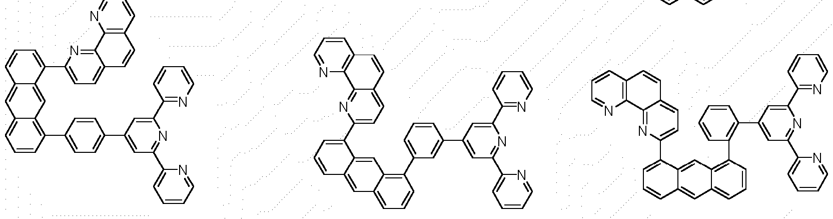

### [Chemical Formula 8]

The compounds of Chemical Formula 8 do not fall under the claimed scope.

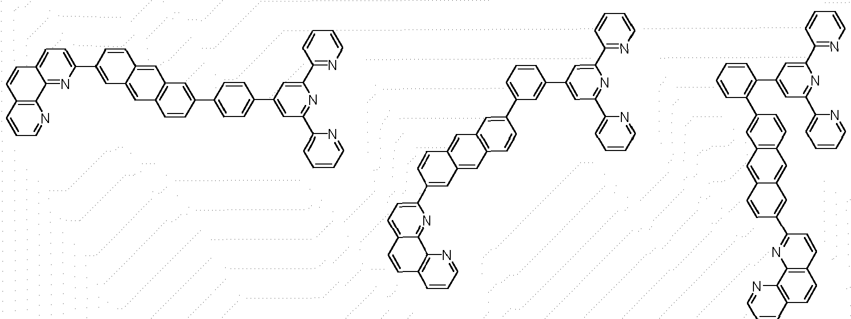

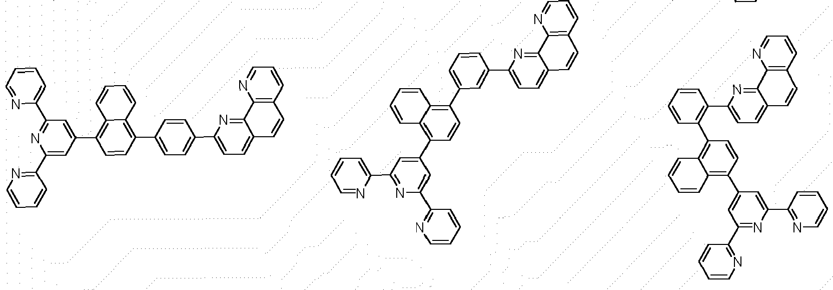

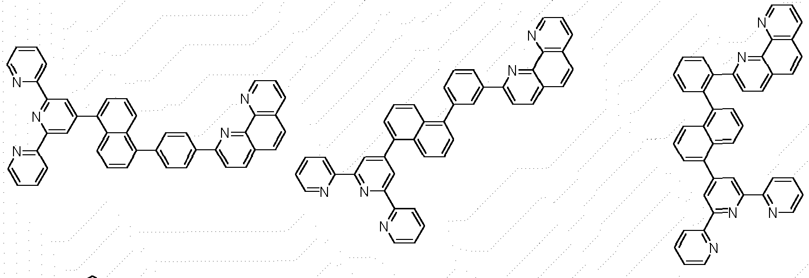

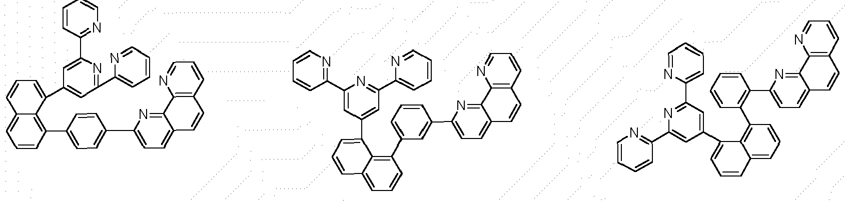

### [Chemical Formula 9]

The compounds of Chemical Formula 9 do not fall under the claimed scope.

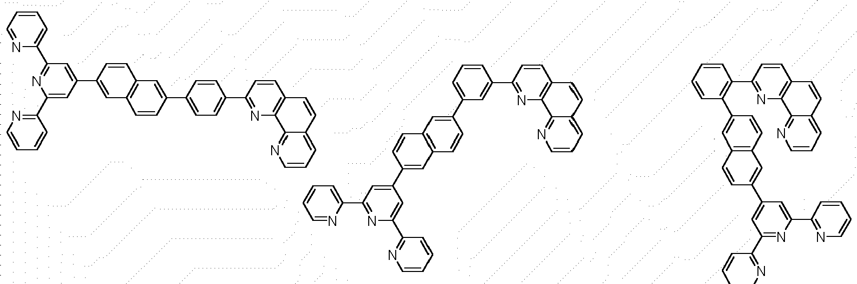

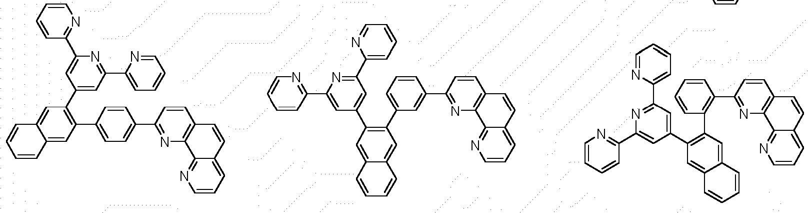

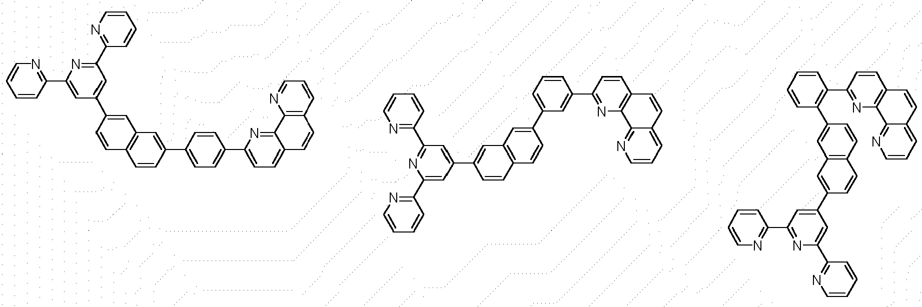

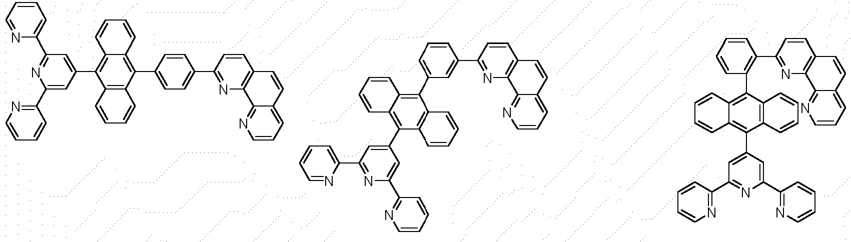

### [Chemical Formula 10]

The compounds of Chemical Formula 10 do not fall under the claimed scope.

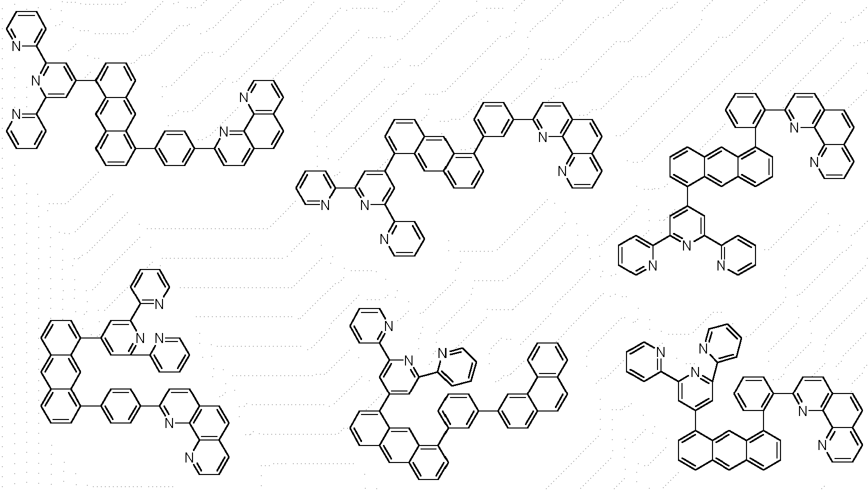

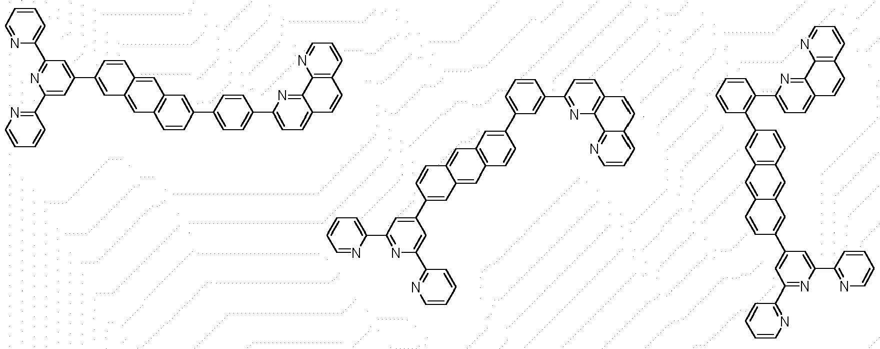

### [Chemical Formula 11]

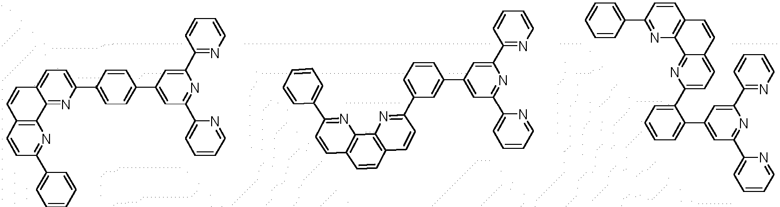

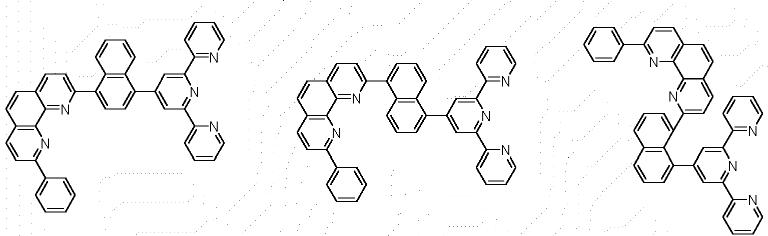

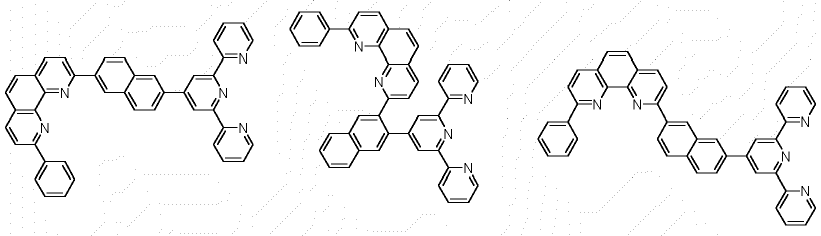

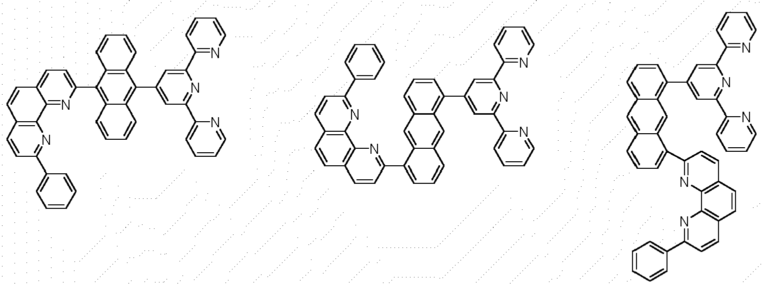

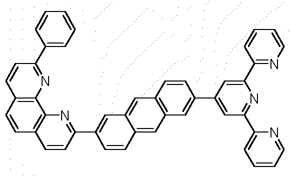

### [Chemical Formula 12]

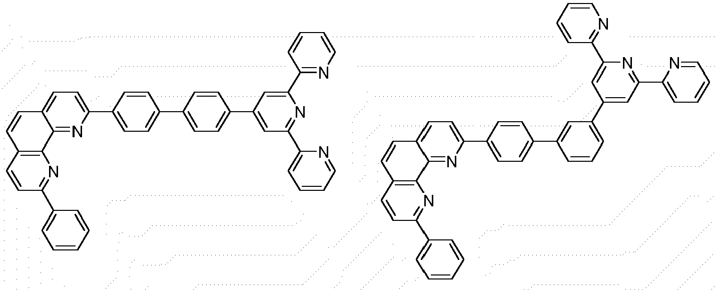

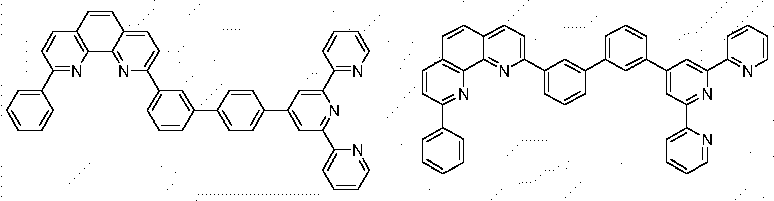

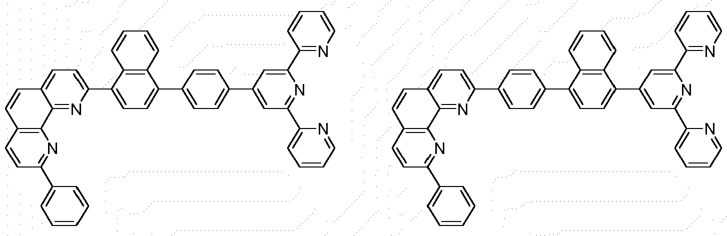

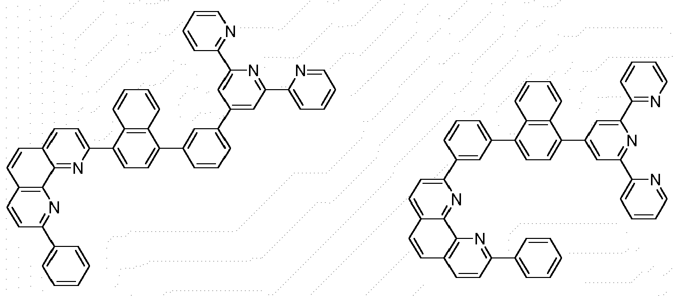

### [Chemical Formula 13]

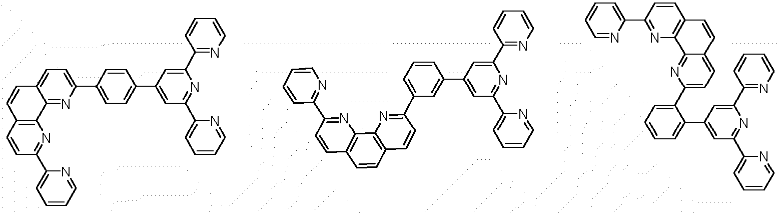

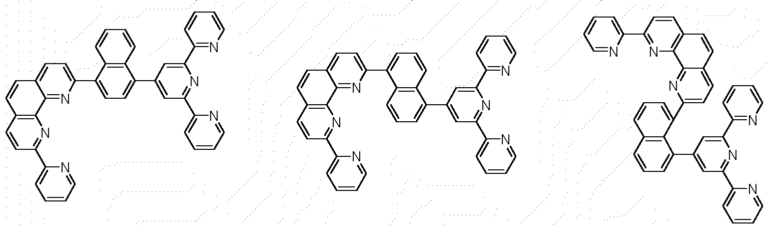

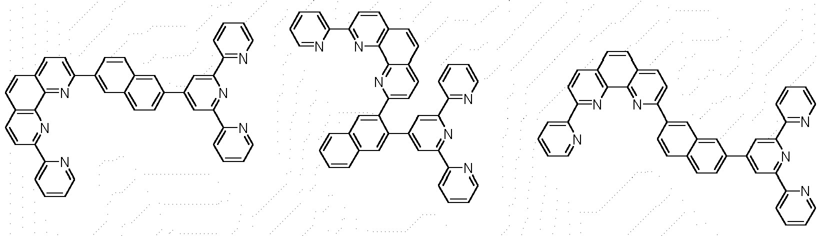

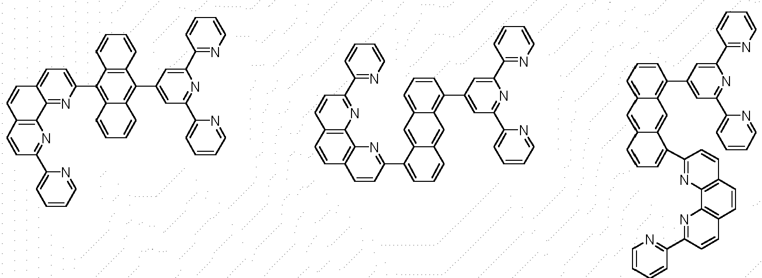

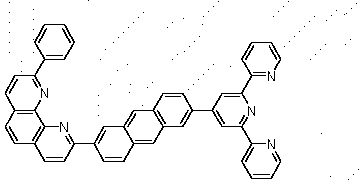

### [Chemical Formula 14]

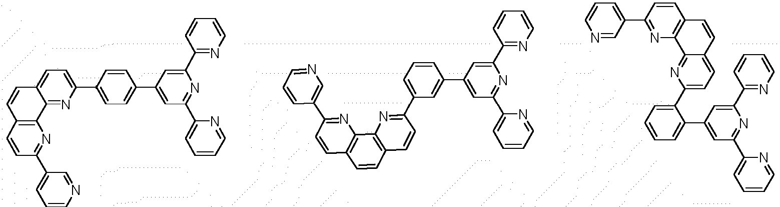

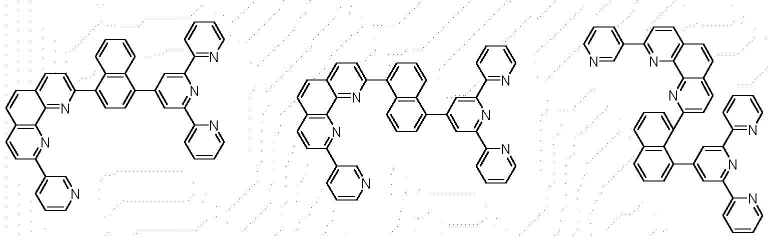

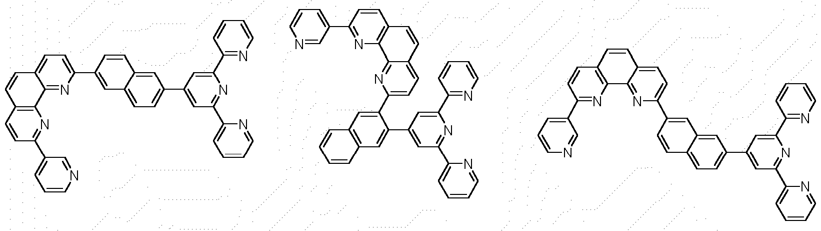

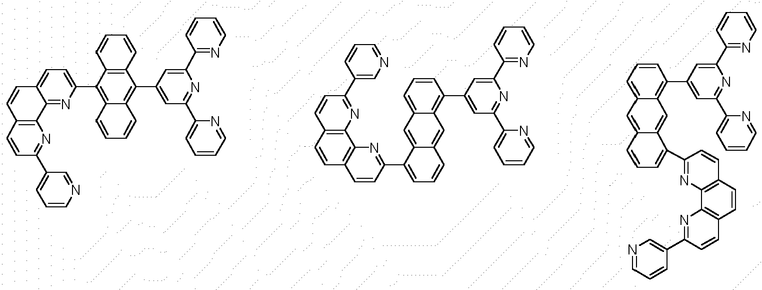

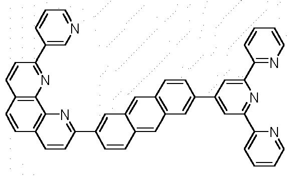

### [Chemical Formula 15]

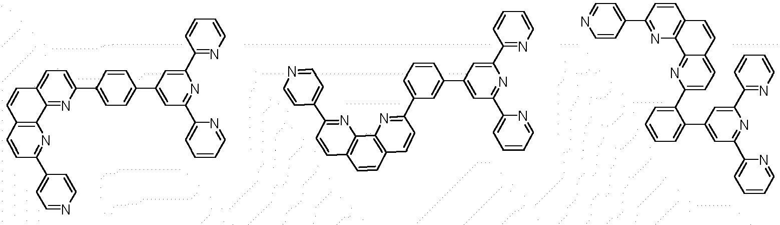

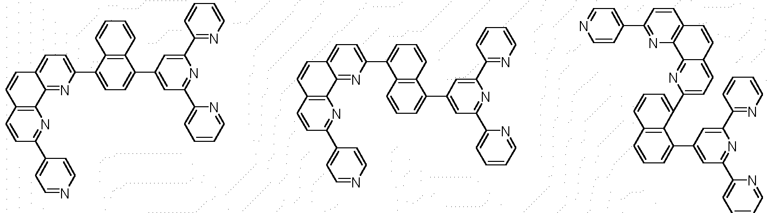

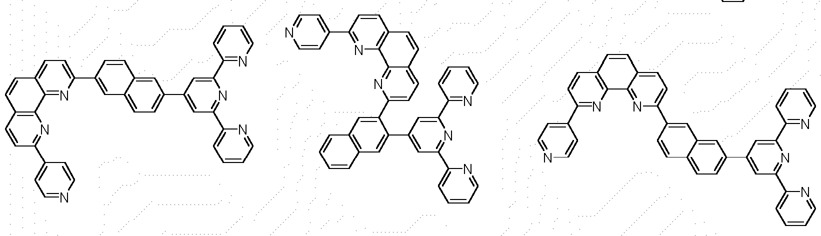

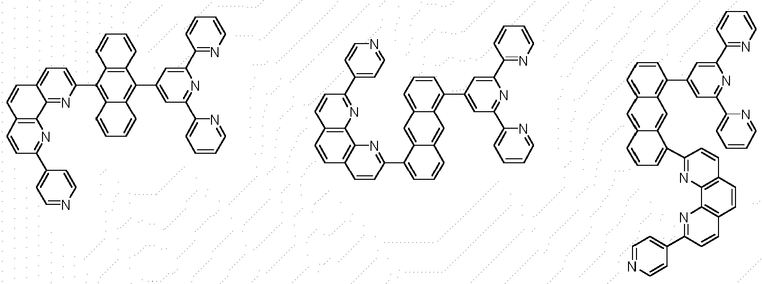

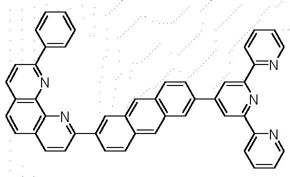

### [Chemical Formula 16]

The compounds of Chemical Formula 16 do not fall under the claimed scope.

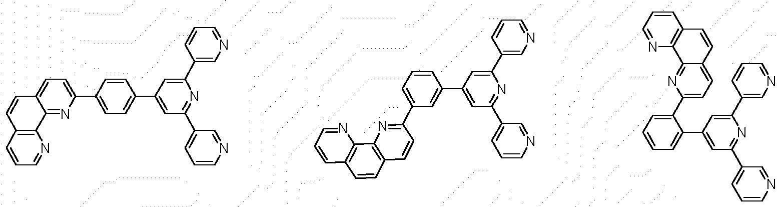

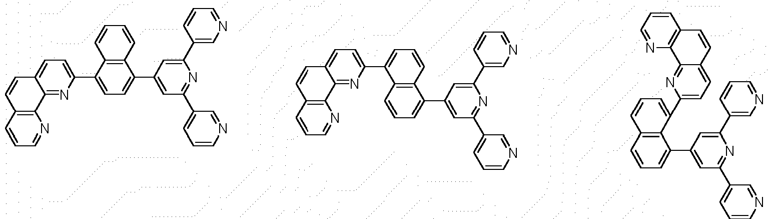

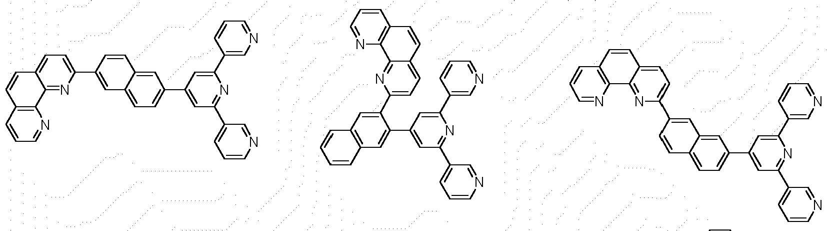

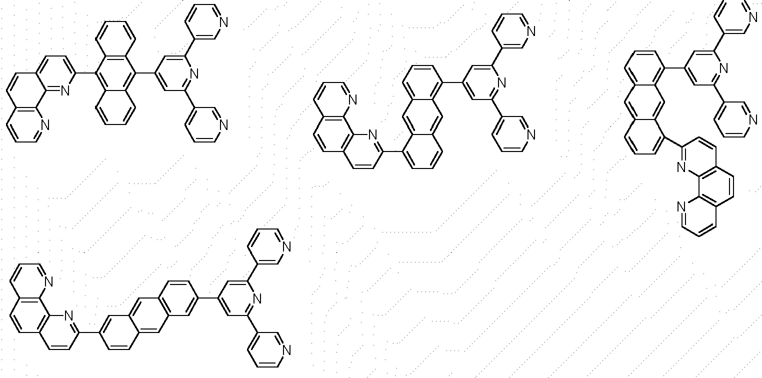

### [Chemical Formula 17]

The compounds of Chemical Formula 17 do not fall under the claimed scope.

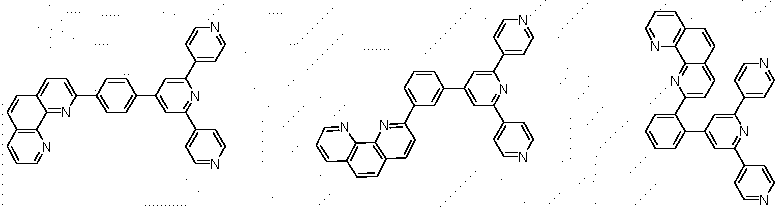

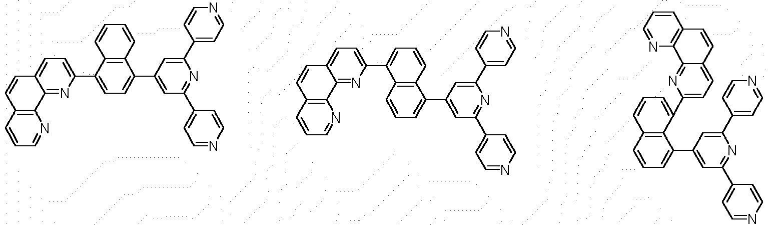

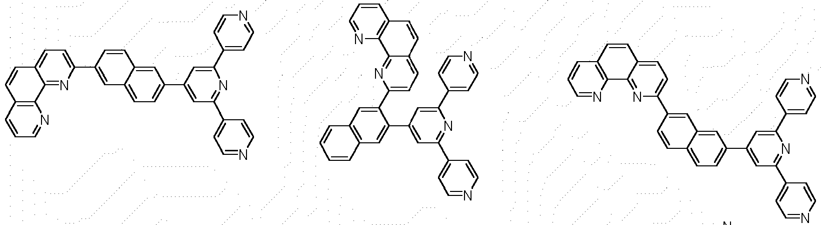

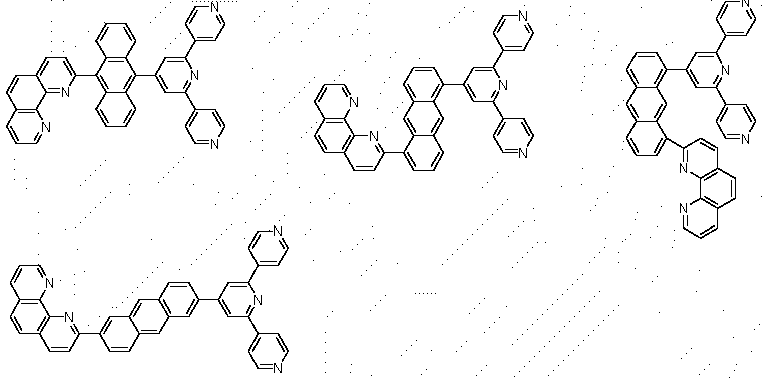

[Chemical Formula 18]The compounds of Chemical Formula 18 do not fall under the claimed scope.

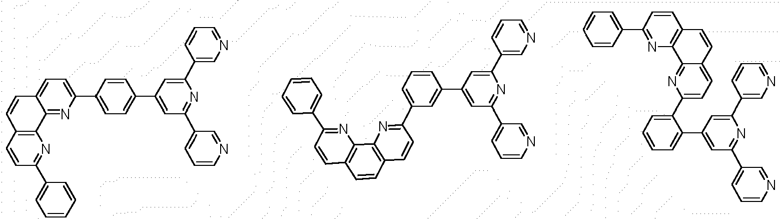

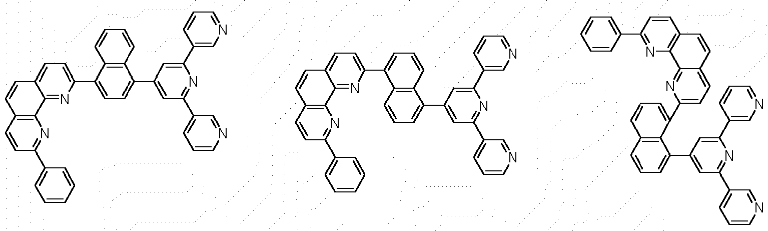

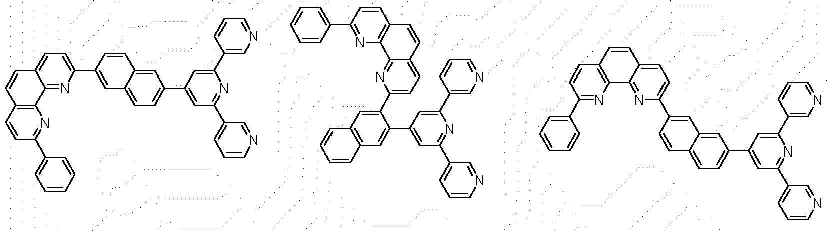

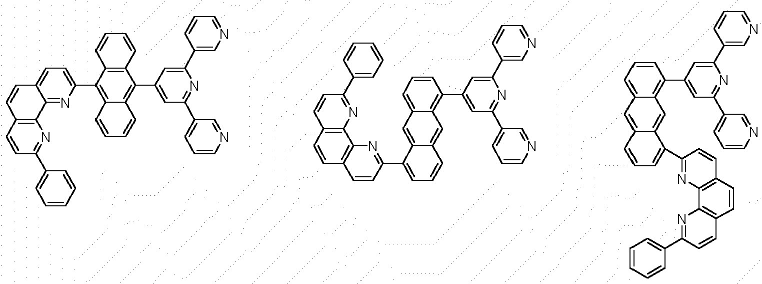

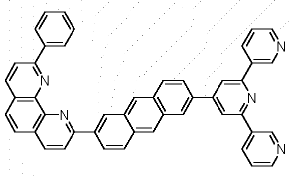

### [Chemical Formula 19]

The compounds of Chemical Formula 19 do not fall under the claimed scope.

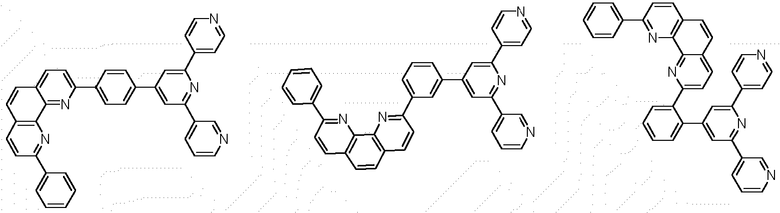

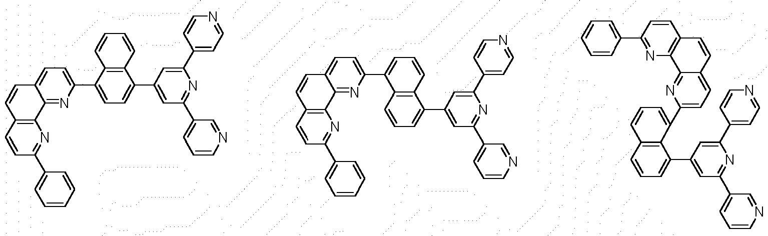

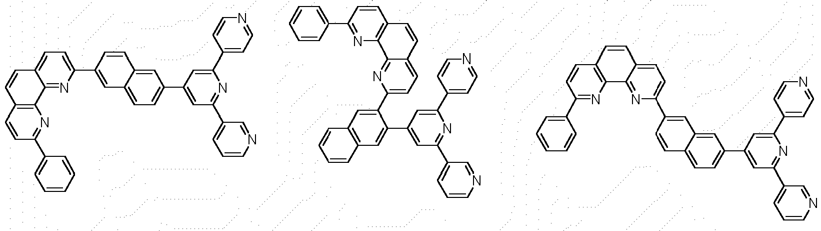

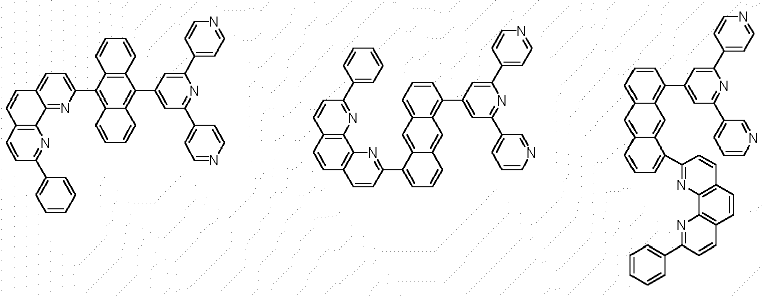

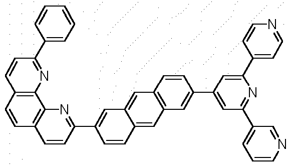

### [Chemical Formula 20]

In Chemical Formula 20, those compounds marked with "*" do not fall under the claimed scope.

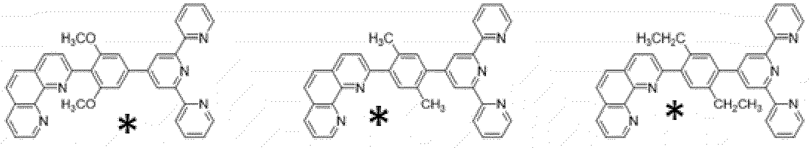

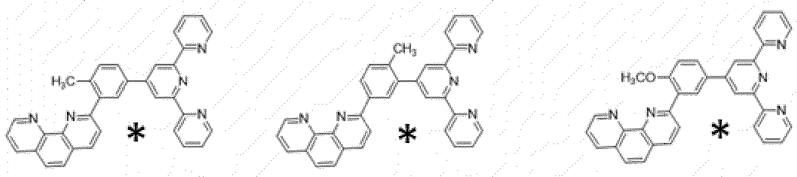

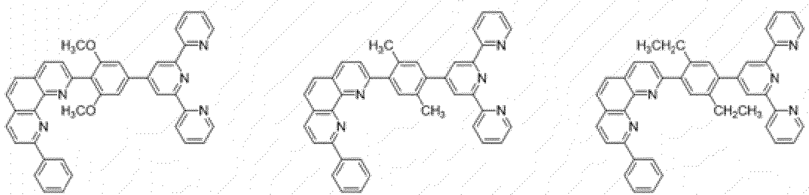

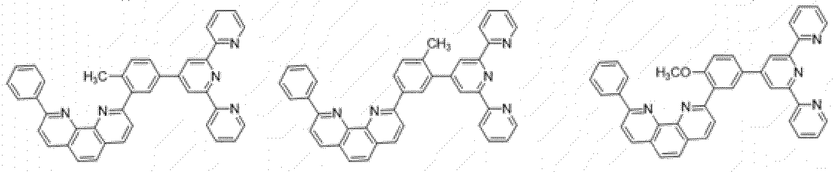

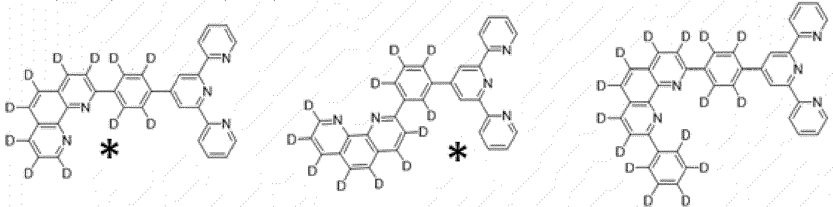

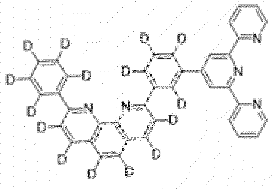

The organic EL element material represented by the general formula (1) can be synthesized by a known synthesis method. Examples of the synthesis method include, but are not limited to, a coupling reaction between an aryl halide derivative and an arylboronic acid derivative using palladium.

The organic EL element material represents a material used for any of the layers constituting the organic EL element. Examples of the layer in which the organic EL element material represented by the general formula (1) is used include, as mentioned later, a hole injection layer, a hole transporting layer, an emissive layer, an electron transporting layer, an electrode protective film (capping layer) and the like. By using the material represented by the general formula (1) in the present invention for any of the layers of the organic EL element, it is possible to provide an organic EL element excellent in luminance efficiency and durability life.

### (Organic EL Element)

Embodiments of the light emitting element of the present invention will be described in detail below. The light emitting element of the present invention includes an anode and a cathode, and an organic layer existing between the anode and the cathode, and the organic layer emits light from electric energy.

Examples of the layer constitution between the anode and the cathode in such organic EL element include, in addition to the constitution composed only of an emissive layer, 1) emissive layer/electron transporting layer, 2) hole transporting layer/emissive layer, 3) hole transporting layer/emissive layer/electron transporting layer, 4) hole injection layer/hole transporting layer/emissive layer/electron transporting layer, 5) hole transporting layer/emissive layer/electron transporting layer/electron injection layer, 6) hole injection layer/hole transporting layer/emissive layer/electron transporting layer/electron injection layer, and 7) hole injection layer/hole transporting layer/emissive layer/hole blocking layer/electron transporting layer/electron injection layer.

Furthermore, the layer constitution may be a tandem type in which a plurality of layers are laminated via an intermediate layer. The intermediate layer is also generally referred to as an intermediate electrode, an intermediate conductive layer, a charge generation layer, an electron withdrawing layer, a connection layer or an intermediate insulating layer, and known material constitutions can be used. Specific examples of the tandem type include lamination constitutions in which a charge generation layer is included as the intermediate layer between the anode and the cathode, such as 8) hole transporting layer/emissive layer/electron transporting layer/charge generation layer/hole transporting layer/emissive layer/electron transporting layer, and 9) hole injection layer/hole transporting layer/emissive layer/electron transporting layer/electron injection layer/charge generation layer/hole injection layer/hole transporting layer/emissive layer/electron transporting layer/electron injection layer.

Each of the above layers may be either a single layer or a plurality of layers, and may be doped. In particular, when the electron injection layer and the charge generation layer are metal-doped layers doped with metal, the electron transportation capability and the electron injection capability to other adjacent layers can be improved, which is preferable. It is preferable to further include, in addition to the above layers, a protective layer (capping layer) since the luminance efficiency can be further improved by the optical interference effect.

The organic EL element material represented by the general formula (1) may be used for any of the above layers in the organic EL element, but is particularly preferably used for the electron transporting layer, charge generation layer or electron injection layer. The constitution of the organic EL element of the present invention is preferably the constitution in which at least an electron transporting layer and an emissive layer are included between an anode and a cathode, and the electron transporting layer contains the organic EL element material represented by general formula (1), the constitution in which at least a charge generation layer and an emissive layer are included between an anode and a cathode, and the charge generation layer contains the organic EL element material represented by the general formula (1), or the constitution in which at least an electron injection layer and an emissive layer are included between an anode and a cathode, and the electron injection layer contains the organic EL element material represented by the general formula (1). Of these layers, two or more layers may contain the organic EL element material represented by the general formula (1).

In the organic EL element according to the embodiment of the present invention, the anode and the cathode have a role for supplying a sufficient current for light emission of the element. It is desirable that at least one of them is transparent or translucent in order to take out light from the light emitting element. Usually, the anode formed on a substrate is made to be a transparent electrode.

### (Substrate)

In order to maintain the mechanical strength of the organic EL element, the organic EL element is preferably formed on a substrate. Examples of the substrate include a glass substrate made of soda glass or alkali-free glass, a plastic substrate and the like. When using the glass substrate, the thickness of the glass substrate has a sufficient thickness for maintaining the mechanical strength and 0.5 mm or more is sufficient. Regarding the material of glass, it is preferable that fewer ions are eluted from glass, and the alkali-free glass is preferable. Soda lime glass provided with a barrier coating such as SiO₂ is commercially available and can also be used.

### (Anode)

The anode is formed on the substrate. The material used for the anode is preferably a material capable of efficiently injecting holes into the organic layer. The material is preferably transparent or translucent so as to take out light. Examples of a material used in the anode include electrically conductive metal oxides such as zinc oxide, tin oxide, indium oxide, indium tin oxide (ITO) and indium zinc oxide (IZO); metals such as gold, silver and chromium; inorganic electrically conductive substances such as copper iodide and copper sulfide; and electrically conductive polymers such as polythiophene, polypyrrole and polyaniline. Of these, ITO glass or Nesa glass are preferable. These electrode materials may be used alone, or a plurality of materials may be used by lamination or mixing. The electrical resistance of the substrate on which the anode is formed may be within a range in which a current sufficient for light emission of the device can be supplied, but low resistance is preferable from the viewpoint of power consumption of the element. For example, an ITO substrate having 300 Ω/□ or less functions as an element electrode. Currently, it has become possible to supply a current to a substrate having about 10 Ω/□, so that it is particularly preferable to use a substrate having low resistance of 20 Ω/□ or less. The thickness of the anode can be arbitrarily selected according to the resistance value, and is usually used in a thickness between 45 to 300 nm in many cases.

### (Hole Injection Layer)

The hole injection layer is a layer inserted between the anode and the hole transporting layer. The hole injection layer may be a single layer or a laminate of a plurality of layers. The presence of a hole injection layer between the hole transporting layer and the anode is preferable since it not only enables the device to be driven at lower voltage and extends the durability life, but also improves the carrier balance of the element and the luminance efficiency.

Examples of the material used for the hole injection layer include, but are not particularly limited to, benzidine derivatives such as 4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB) and bis(N,N'-diphenyl-4-aminophenyl)-N,N-diphenyl-4,4'-diamino-1,1'-biphenyl (TPD232); group of materials called starburst arylamines such as 4,4',4"-tris(3-methylphenyl(phenyl)amino)triphenylamine (m-MTDATA) and 4,4',4"-tris(1-naphthyl(phenyl)amino)triphenylamine (1-TNATA); triarylamine derivatives; biscarbazole derivatives such as bis(N-arylcarbazole) and bis(N-alkylcarbazole); pyrazoline derivatives; stilbene-based derivetives; hydrazone-based compounds; heterocyclic compounds such as benzofuran derivatives, thiophene derivatives, oxadiazole derivatives, phthalocyanine derivatives and porphyrin derivatives; polycarbonates and styrene derivatives having the above monomers in side chains; polymer-based materials such as polythiophene, polyaniline, polyfluorene, polyvinylcarbazole and polysilane. From the viewpoint of smoothly injecting and transporting holes from the anode to the hole transporting layer, the benzidine derivatives or group of starburst arylamine-based materials are more preferably used.

These materials may be used alone, or two or more materials may be used by mixing. A hole injection layer may be formed by laminating a plurality of materials. Furthermore, it is more preferable that the hole injection layer is composed of an acceptor compound alone, or the hole injection material as mentioned above doped with an acceptor compound is used since the above effects can be obtained more remarkably. The acceptor compound is a material which forms a charge-transfer complex with a contacted hole transporting layer when used as a single-layer film, or a material which forms a charge-transfer complex with a material constituting a hole injection layer when doped for use. The use of such a material improves the conductivity of the hole injection layer and further contributes to a reduction in driving voltage of the element, thus enabling further improvement in luminance efficiency and durability life.

Examples of the acceptor compound include metal chlorides such as iron(III) chloride, aluminum chloride, gallium chloride, indium chloride and antimony chloride; metal oxides such as molybdenum oxide, vanadium oxide, tungsten oxide and ruthenium oxide; charge-transfer complexes such as tris(4-bromophenyl)aminium hexachloroantimonate (TBPAH); organic compounds having a nitro group, a cyano group, halogen or a trifluoromethyl group in the molecule; quinone-based compounds; acid anhydride-based compounds; fullerenes and the like. Of these, metal oxides and cyano group-containing compounds are preferable since it is easy to handle and easy to be deposited, thus easily obtaining the above effects. In either case where the hole injection layer is composed of an acceptor compound alone or where the hole injection layer is doped with an acceptor compound, the hole injection layer may be a single layer or may be composed of a plurality of layers by lamination.

### (Hole Transporting Layer)

The hole transporting layer is a layer which transports holes injected from the anode to the emissive layer. The hole transporting layer may be a single layer or may be composed of a plurality of layers by lamination.

Examples of the material used for the hole transporting layer include those exemplified as materials used for the hole injection layer. From the viewpoint of smoothly injecting and transporting holes into the emissive layer, the triarylamine derivatives or benzidine derivative are more preferable.

### (Emissive Layer)

The emissive layer may be either a single layer or a plurality of layers. The emissive material is composed of an emissive material and may be a mixture of a host material and a dopant material, a host material alone, or a mixture of two host materials and one dopant material. Namely, in the organic EL element according to the embodiment of the present invention, only the host material or the dopant material may emit light, or both the host material and the dopant material may emit light in each emissive layer. From the viewpoint of efficiently utilizing electric energy and obtaining light emission with high color purity, the emissive layer is preferably composed of a mixture of the host material and the dopant material. The host material and the dopant material may be alone or a combination of a plurality thereof, respectively. When the emissive layer is composed of a mixture of the host material and the dopant material, the dopant material may be contained in a whole host material, or may be partially contained therein. The dopant material may be either laminated or dispersed. The dopant material can control a luminescent color. From the viewpoint of suppressing the concentration quenching phenomenon, the amount of the dopant material is preferably 30% by weight or less, and more preferably 20% by weight or less, based on 100% by weight of the total of the host material and the dopant material. In the doping method, the dopant material can be co-deposited with the host material, or the dopant material may be mixed with the host material in advance, followed by co-deposition.

Specific examples of the emissive material include fused ring derivatives such as anthracene and pyrene, which are known as light emitters; metal chelated oxynoid compounds such as tris(8-quinolinolate)aluminum; bisstyryl derivatives such as bisstyrylanthracene derivatives and distyrylbenzene derivatives; tetrapthenylbutadiene derivatives, indene derivatives, coumarine derivatives, oxadiazole derivatives, pyrrolopyridine derivatives, perinone derivatives, cyclopentadiene derivatives, oxadiazole derivatives, thiadiazolopyridine derivatives, dibenzofuran derivatives, carbazole derivatives and indolocarbazole derivatives; and polymers such as polyphenylenevinylene derivatives, polyparaphenylene derivatives and polythiophene derivatives.

The host material contained in the emissive material is not necessarily limited to the compound alone, and a plurality of compounds may be used by mixing or lamination. Examples of the host material include, but are not particularly limited to, compounds having a fused aryl ring such as naphthalene, anthracene, phenanthrene, pyrene, chrysene, naphthacene, triphenylene, perylene, fluoranthene, fluorene and indene, and derivatives thereof; aromatic amine derivatives such as N,N'-dinaphthyl-N,N'-diphenyl-4,4'-diphenyl-1,1'-diamine; metal chelated oxynoid compounds such as tris(8-quinolinato)aluminum(III); bisstyryl derivatives such as distyrylbenzene derivatives; tetraphenylbutadiene derivatives, indene derivatives, coumarine derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives and triazine derivatives; and polymers such as polyphenylenevinylene derivatives, polyparaphenylene derivatives, polyfluorene derivatives, polyvinylcarbazole derivatives and polythiophene derivatives. Of these, as a host which is used when the emissive layer performs triplet emission (phosphorescence emission), metal chelated oxynoid compounds, dibenzofuran derivatives, dibenzothiophene derivatives, carbazole derivatives, indolocarbazole derivatives, triazine derivatives and triphenylene derivatives are preferably used.

Examples of the dopant material contained in the emissive material include, but are not particularly limited to, compounds having an aryl ring such as naphthalene, anthracene, phenanthrene, pyrene, fluoranthene, triphenylene, perylene, fluorene and indene, and derivatives thereof (for example, 2-(benzothiazol-2-yl)-9,10-diphenylanthracene and 5,6,11,12-tetraphenylnaphthacene); compounds having a heteroaryl ring such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyrazine, naphthyridine, quinoxaline, pyrrolopyridine and thioxanthene, and derivatives thereof; distyrylbenzene derivatives; aminostyryl derivatives such as 4,4'-bis(2-(4-diphenylaminophenyl)ethenyl)biphenyl, 4,4'-bis(N-(stilben-4-yl)-N-phenylamino)stilbene; aromatic acetylene derivatives, tetraphenylbutadiene derivatives, stilbene derivatives, aldazine derivatives, pyrromethene derivatives, diketopyrrolo[3,4-c]pyrrole derivatives; coumarine derivatives such as 2,3,5,6-1H,4H-tetrahydro-9-(2'-benzothiazolyl)quinolizino[9,9a,1-gh]coumarine; azole derivatives such as imidazole, thiazole, thiadiazole, carbazole, oxazole, oxadiazole and triazole, and metal complexes thereof; and aromatic amine derivatives such as N,N'-diphenyl-N,N'-di(3-methylphenyl)-4,4'-diphenyl-1,1'-diamine; a compound represented by the following general formula (2). Of these, a dopant containing a diamine skeleton and a dopant containing a fluoranthene skeleton can further improve the luminance efficiency, and a compound represented by the following general formula (2) can further improve the luminance efficiency and durability life.

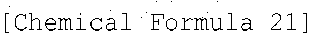

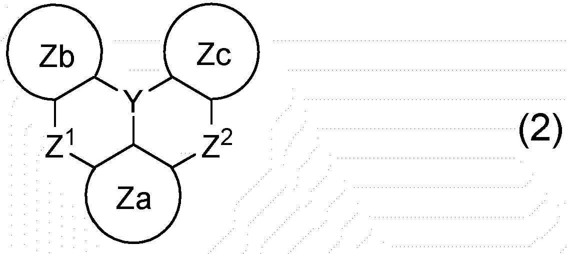

In the general formula (2), a Za ring, a Zb ring and a Zc ring are each independently a substituted or unsubstituted aryl ring having 6 to 30 ring-forming carbon atoms, or a substituted or unsubstituted heteroaryl ring having 5 to 30 ring-forming atoms. Preferably, the Za ring, the Zb ring and the Zc ring are each independently a substituted or unsubstituted aryl ring having 6 to 30 ring-forming carbon atoms. Z¹ and Z² are each independently an oxygen atom, NRa (a nitrogen atom having a substituent Ra) or a sulfur atom, and when Z¹ is NRa, Ra may or may not be linked to the Za ring or the Zb ring to form a ring, and when Z² is NRa, Ra may or may not linked to the Zb ring or the Zc ring to form a ring. Ra is each independently a substituted or unsubstituted aryl group having 6 to 30 ring-forming carbon atoms, a substituted or unsubstituted heteroaryl group having 5 to 30 ring-forming carbon atoms, or a substituted or unsubstituted alkyl group having 1 to 30 carbon atoms. Both Z¹ and Z² are NRa, and Ra is preferably a substituted or unsubstituted aryl group having 6 to 30 ring-forming carbon atoms. Y is a boron atom, a phosphorus atom, SiRb (a silicon atom having a substituent Rb), P = O or P = S. Rb is each independently a substituted or unsubstituted aryl group having 6 to 30 ring-forming carbon atoms, a substituted or unsubstituted heteroaryl group having 5 to 30 ring-forming carbon atoms, or a substituted or unsubstituted alkyl group having 1 to 30 carbon atoms. Y is preferably a boron atom. In all the above groups, when the groups are substituted, the substituent is preferably an alkyl group, a cycloalkyl group, a heterocyclic group, an alkenyl group, a cycloalkenyl group, an alkynyl group, an aryl group, a heteroaryl group, a hydroxyl group, a thiol group, an alkoxy group, an alkylthio group, an arylether group, an arylthio ether group, an halogen, an cyano group, an aldehyde group, an acyl group, a carboxyl group, an ester group, an amide group, an acyl group, a sulfonyl group, a sulfonic acid ester group, a sulfonamide group, an amino group, a nitro group, a silyl group, a siloxanyl group, a boryl group or an oxo group. These substituents may be further substituted with the above substituents.

Examples of the alkyl group and alkoxy group include those exemplified as the substituent in the general formula (1) .

The cycloalkyl group represents, for example, a saturated alicyclic hydrocarbon group such as a cyclopropyl group, a cyclohexyl group, a norbornyl group or an adamantyl group, which may or may not have a substituent. The number of the ring-forming carbon atoms is not particularly limited, but is preferably in a range of 3 or more and 20 or less.

The heterocyclic group represents, for example, an aliphatic ring containing atoms other than the carbon atom in the ring, such as a pyran ring, a piperidine ring or a cyclic amide, which may or may not have a substituent. The number of the ring-forming atom is not particularly limited, but is preferably in a range of 3 or more and 20 or less.

The alkenyl group represents, for example, an unsaturated aliphatic hydrocarbon group containing a double bond, such as a vinyl group, an allyl group or a butadienyl group, which may or may not have a substituent. The number of carbon atoms of the alkenyl group is not particularly limited, but is preferably in a range of 2 or more and 20 or less.

The cycloalkenyl group represents, for example, an unsaturated alicyclic hydrocarbon group containing a double bond, such as a cyclopentenyl group, a cyclopentadienyl group or a cyclohexenyl group, which may or may not have a substituent.

The alkynyl group represents, for example, an unsaturated aliphatic hydrocarbon group containing a triple bond, such as an ethynyl group, which may or may not have a substituent. The number of carbon atoms of the alkynyl group is not particularly limited, but is preferably in a range of 2 or more and 20 or less.

The aryl group represents, for example, aromatic hydrocarbon groups such as a phenyl group, a biphenyl group, a terphenyl group, a naphthyl group, a fluorenyl group, a benzofluorenyl group, a dibenzofluorenyl group, a phenanthryl group, an anthracenyl group, a benzophenanthryl group, a benzoanthracenenyl group, a chrysenyl group, a pyrenyl group, a fluoranthenyl group, a triphenylenyl group, a benzofluoranthenyl group, a dibenzoanthracenyl group, a perylenyl group or a helicenyl group. Of these, a phenyl group, a biphenyl group, a terphenyl group, a naphthyl group, a fluorenyl group, a phenanthryl group, an anthracenyl group, a pyrenyl group, a fluoranthenyl group and a triphenylenyl group are preferable. The aryl group may or may not have a substituent. The number of carbon atoms of the aryl group is not particularly limited, but is preferably in a range of 6 or more and 40 or less, and more preferably 6 or more and 30 or less.

In the substituted phenyl group, when there are substituents on two adjacent carbon atoms in the phenyl group, the substituents may form a ring structure. The resulting group can correspond to one or more of "substituted phenyl group", "aryl group having a structure in which two or more rings are fused" and "heteroaryl group having a structure in which two or more rings are fused" depending on the structure thereof.

The heteroaryl group represents, for example, a cyclic aromatic group having one atom or a plurality of atoms other than the carbon atom in the ring, such as a pyridyl group, a furanyl group, a thiophenyl group, a quinolinyl group, an isoquinolinyl group, a pyrazinyl group, a pyrimidyl group, a pyridazinyl group, a triazinyl group, a napthyridinyl group, a cinnolinyl group, a phthalazinyl group, a quinoxalinyl group, a quinazolinyl group, a benzofuranyl group, a benzothiophenyl group, an indolyl group, a dibenzofuranyl group, a dibenzothiophenyl group, a carbazolyl group, a benzocarbazolyl group, a carbolinyl group, an indolocarbazolyl group, a benzoflocarbazolyl group, a benzothienocarbazolyl group, a dihydroindenocarbazolyl group, a benzoquinolinyl group, an acridinyl group, a dibenzoacridinyl group, a benzimidazolyl group, an imidazopyridyl group, a benzoxazolyl group, a benzothiazolyl group or a phenanthrolinyl group. However, the naphthyridinyl group represents any of a 1,5-naphthyridinyl group, a 1,6-naphthyridinyl group, a 1,7-naphthyridinyl group, a 1,8-naphthyridinyl group, a 2,6-naphthyridinyl group and a 2,7-naphthyridinyl group. The heteroaryl group may or may not have a substituent. The number of the ring-forming atom of the heteroaryl group is not particularly limited, but is preferably in a range of 3 or more 40 or less, and more preferably 3 or more 30 or less.

The alkylthio group is a group in which an oxygen atom of an ether bond of an alkoxy group is substituted with a sulfur atom. The alkylthio group may or may not have a substituent. The number of carbon atoms of the alkylthio group is not particularly limited, but is preferably in a range of 1 or more and 20 or less.

The arylether group represents, for example, a functional group to which an aromatic hydrocarbon group is linked via an ether bond, such as a phenoxy group, and the aromatic hydrocarbon group may or may not have a substituent. The number of carbon atoms of the arylether group is not particularly limited, but is preferably in a range of 6 or more and 40 or less.

The arylthioether group is a group in which an oxygen atom of an ether bond of an arylether group is substituted with a sulfur atom, which may or may not have a substituent. The number of carbon atoms of the alkylthioether group is not particularly limited, but is preferably in a range of 6 or more 40 or less.

The halogen represents fluorine, chlorine, bromine or iodine.

The acyl group represents, for example, a functional group in which an alkyl group, a cycloalkyl group, an alkenyl group, an alkynyl group, an aryl group or a heteroaryl group is linked via a carbonyl group, such as an acetyl group, a propionyl group, a benzoyl group or an acrylyl group, which may or may not have a substituent. The number of carbon atoms of the acyl group is not particularly limited, but is preferably 2 or more and 40 or less, and more preferably 2 or more and 30 or less.

The ester group represents, for example, a functional group in which an alkyl group, a cycloalkyl group, an aryl group or a heteroaryl group is linked via an ester bond, which may or may not have a substituent. The number of carbon atoms of the ester group is not particularly limited, but is preferably in a range of 1 or more 20 or less. More specifically, examples thereof include a methyl ester group such as a methoxycarbonyl group, an ethyl ester group such as an ethoxycarbonyl group, a propyl ester group such as a propoxycarbonyl group, a butyl ester group such as a butoxycarbonyl group, an isopropyl group such as an isopropoxymethoxycarbonyl group, a hexyl ester group such as a hexyloxycarbonyl group, and a phenyl ester group such as a phenoxycarbonyl group.

The amide group represents, for example, a functional group in which an alkyl group, a cycloalkyl group, an aryl group or a heteroaryl group is linked via an amide bond, which may or may not have a substituent. The number of carbon atoms of the amide group is not particularly limited, but is preferably in a range of 1 or more and 20 or less. More specifically, examples thereof include a methylamido group, an ethylamido group, a propylamide group, a butylamide group, an isopropylamide group, a hexylamide group, a phenylamido group and the like.

The sulfonyl group represents, for example, a functional group in which an alkyl group, a cycloalkyl group, an aryl group or a heteroaryl group is linked via a -S(=O)₂-bond, which may or may not have a substituent. The number of carbon atoms of the sulfonyl group is not particularly limited, but is preferably in a range of 1 or more 20 or less.

The sulfonic acid ester group represents, for example, a functional group in which an alkyl group, a cycloalkyl group, an aryl group or a heteroaryl group is linked via a sulfonic acid ester bond, which may or may not have a substituent. As used herein, the sulfonic acid ester bond refers to those in which the carbonyl moiety of the ester bond, that is, -C(=O)- is substituted with the sulfonyl moiety, that is, -S(=O)₂-. The number of carbon atoms of the sulfonic acid ester group is not particularly limited, but is preferably in a range of 1 or more and 20 or less.

The sulfonamide group represents, for example, a functional group in which an alkyl group, a cycloalkyl group, an aryl group or a heteroaryl group is linked via a sulfonamide bond, which may or may not have a substituent. As used herein, the sulfonamide bond refers to those in which the carbonyl moiety of the amide bond, that is, -C(=O)- is substituted with the sulfonyl moiety, that is, -S(=O)₂-. The number of carbon atoms in the sulfonamide group is not particularly limited, but is preferably in a range of 1 or more and 20 or less.

The amino group may or may not have a substituent. The number of carbon atoms of the amino group is not particularly limited, but is preferably in a range of 2 or more and 50 or less, more preferably 6 or more and 40 or less, and particularly preferably 6 or more and 30 or less.

The silyl group represents a functional group to which a substituted or unsubstituted silicon atom is linked, and examples thereof include an alkylsilyl group such as a trimethylsilyl group, a triethylsilyl group, a tertbutyldimethylsilyl group, a propyldimethylsilyl group or a vinyldimethylsilyl group, and an arylsilyl group such as a phenyldimethylsilyl group, a tert-butyldiphenylsilyl group, a triphenylsilyl group or a trinaphthylsilyl group. The silyl group may or may not have a substituent. The number of carbon atoms in the silyl group is not particularly limited, but is preferably in a range of 1 or more and 30 or less.

The siloxanyl group represents, for example, a silicon compound group via an ether bond such as a trimethylsiloxanyl group. The siloxanyl group may or may not have a substituent.

The boryl group may or may not have a substituent.

Examples of the compound represented by the general formula (2) include the following examples.

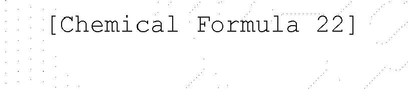

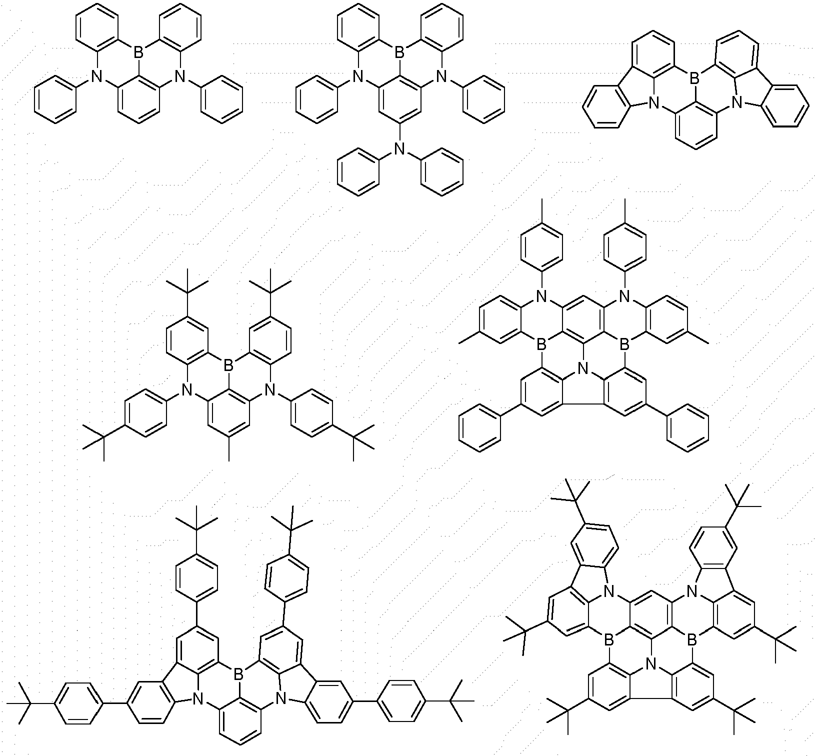

In the organic EL element according to the embodiment of the present invention, it is also preferable that the emissive layer contains a triplet emissive material.

The dopant used when the emissive layer performs triplet emission (phosphorescence emission) is preferably a metal complex compound containing at least one metal selected from the group consisting of iridium (Ir), ruthenium (Ru), palladium (Pd), platinum (Pt), osmium (Os), and rhenium (Re). The ligand constituting the metal complex compound preferably has a nitrogen-containing an aromatic heterocyclic ring such as a phenylpyridine skeleton, a phenylquinoline skeleton or a carbene skeleton. However, the complex is not limited thereto, and a suitable complex is selected in context with luminescent color, element performance and host compound to be required. Specific examples thereof include a tris(2-phenylpyridyl)iridium complex, a tris{2-(2-thiophenyl)pyridyl}iridium complex, a tris{2-(2-benzothiophenyl)pyridyl}iridium complex, a tris(2-phenylbenzothiazole)iridium complex, a tris(2-phenylbenzoxazole)iridium complex, a trisbenzoquinolineiridium complex, a bis(2-phenylpyridyl) (acetylacetonate)iridium complex, a bis{2-(2-thiophenyl)pyridyl}iridium complex, a bis{2-(2-benzothiophenyl)pyridyl}(acetylacetonate)iridium complex, a bis(2-phenylbenzothiazole) (acetylacetonate)iridium complex, a bis(2-phenylbenzoxazole) (acetylacetonate)iridium complex, a bisbenzoquinoline(acetylacetonate)iridium complex, a bis{2-(2,4-difluorophenyl)pyridyl}(acetylacetonate)iridium complex, a tetraethylporphyrin platinum complex, a {tris(senoyltrifluoroacetone)mono(1,10-phenanthroline) }europium complex, a{tris(senoyltrifluoroacetone)mono(4,7-diphenyl-1,10-phenanthroline) }europium complex, a {tris(1,3-diphenyl-1,3-propanedione)mono (1, 10-phenanthroline) }europium complex, a trisacetylacetoneterbium complex and the like. A phosphorescent dopant mentioned in JP 2009-130141 A is also preferably used. An iridium complex or a platinum complex is preferable and can further improve the luminance efficiency.

Each of the above triplet emissive materials used as dopant materials may be contained in the emissive layer alone, or two or more thereof may be used by mixing. When two or more triplet emissive materials are used, the total weight of the dopant materials is preferably 30% by weight or less, and more preferably 20% by weight or less, when the total weight of the host material and the dopant materials is 100% by weight.

Specific examples of preferred host material and dopant material in the triplet emission system include, but are not particularly limited to, the following examples.

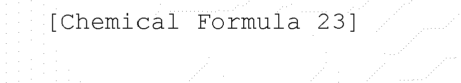

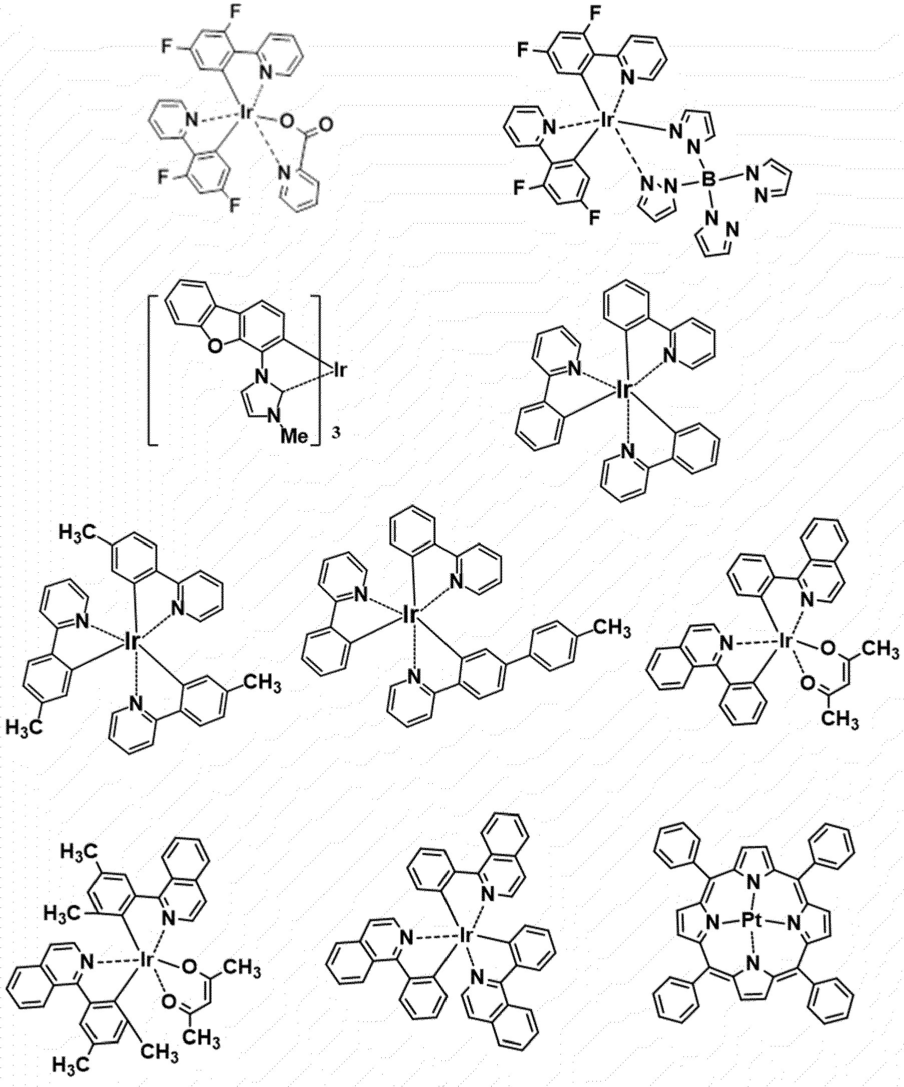

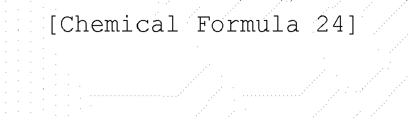

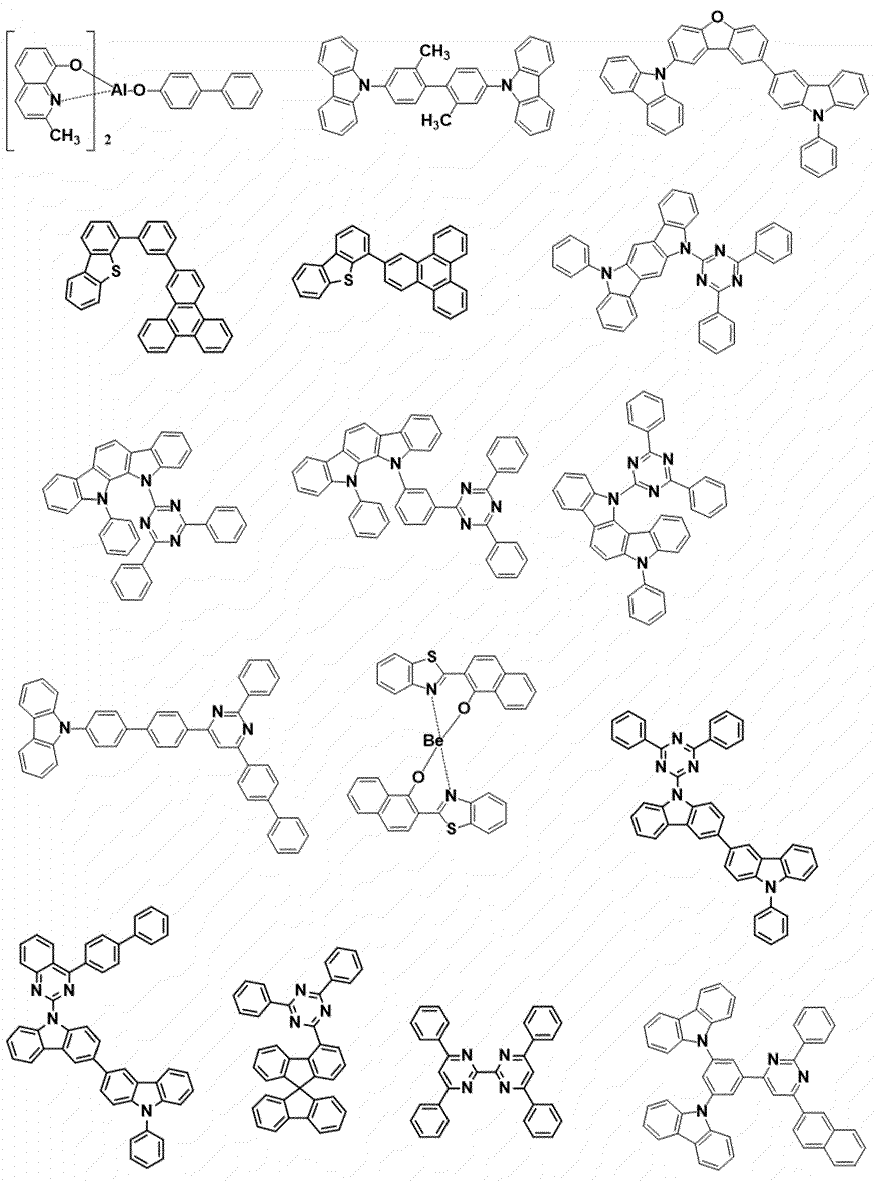

It is also preferable that the emissive layer contains a thermally activated delayed fluorescent material. The thermally activated delayed fluorescence is explained on pages 87 to 103 of "State-of-the-Art Organic EL" (edited by Chihaya Adachi and Hiroshi Fujimoto, published by CMC Publishing Co., Ltd.). In that literature, it is explained that, by bringing the energy levels of an excited singlet state and an excited triplet state close to each other, reverse energy transfers from the excited triplet state, which usually has low transition probability, to the excited singlet state with high efficiency to develop thermally activated delayed fluorescence (TADF). Furthermore, the generation mechanism of delayed fluorescence is explained in FIG. 5 of the literature. Light emission of the delayed fluorescence can be confirmed by transient photo luminescence (PL) measurement.

The thermally activated delayed fluorescent material is also generally referred to as a TADF material. The thermally activated delayed fluorescent material may be a single material which exhibits thermally activated delayed fluorescence, or a plurality of materials which exhibit thermally activated delayed fluorescence. When a plurality of materials are used, they may be used as a mixture, or may be used by laminating layers of each material. A known material can be used as the thermally activated delayed fluorescent material. Examples thereof include, but are not limited to, benzonitrile derivatives, triazine derivatives, disulfoxide derivatives, carbazole derivatives, indolocarbazole derivatives, dihydrophenazine derivatives, thiazole derivatives, oxadiazole derivatives and the like.

It is preferable that the element in which the TADF material is contained in the emissive layer further contains a fluorescent dopant in the emissive layer. This is because triplet excitons are converted into singlet excitons by the TADF material, and the singlet excitons are received by the fluorescent dopant, thus making it possible to achieve higher luminance efficiency and longer durability life.

### (Electron Transporting Layer)

In the present invention, the electron transporting layer is a layer into which electrons are injected from the cathode and which transports the electrons. The electron transporting layer is required to have high electron injection efficiency and efficiently transport injected electrons. Therefore, the material constituting the electron transporting layer is preferably a substance which has large electron affinity, high electron mobility and excellent stability and is less likely to generate impurities acting as a trap during production and use. Particularly, when layers are laminated in a large thickness, since a low-molecular compound is likely to be crystallized to cause deterioration of film quality, a compound having a molecular weight of 400 or more is preferably used so as to maintain stable film quality. However, considering transportation balance between holes and electrons, if the electron transporting layer mainly plays a role of efficiently inhibiting holes from flowing toward the cathode from the anode without being recombined, there is exerted the same effect of improving luminance efficiency as that in the case where the electron transporting layer is composed of a material having high electron transportation capability even if the electron transporting layer is composed of a material having not so high electron transportation capability. Therefore, the electron transporting layer in the present invention also includes a hole blocking layer capable of efficiently blocking the movement of holes. The hole blocking layer and the electron transporting layer may be composed of a single material or a laminate of a plurality of materials.

Examples of electron transporting materials used in the electron transporting layer include fused polycyclic aromatic derivatives such as naphthalene and anthracene; styryl-based aromatic ring derivatives such as 4,4'-bis(diphenylethenyl)biphenyl; quinone derivatives such as anthraquinone and diphenoquinone; phosphorus oxide derivatives; quinolinol complexes such as tris(8-quinolinolato)aluminum(III), benzoquinolinol complexes, hydroxyazole complexes, azomethine complexes, tropolone metal complexes, and various metal complexes such as flavonol metal complexes. It is preferable to use a compound having a heteroaryl ring structure, which is composed of an element selected from carbon, hydrogen, nitrogen, oxygen, silicon and phosphorus and contains an electron-accepting nitrogen, since the driving voltage is more reduced and more efficient light emission is obtained.

The electron-accepting nitrogen as used herein represents a nitrogen atom which forms a multiple bond with an adjacent atom. Since the nitrogen atom has high electronegativity, the multiple bond has electron-accepting property. Therefore, an aromatic heterocycle containing electron-accepting nitrogen has high electron affinity. The electron transporting material having electron-accepting nitrogen makes it easier to accept electrons from the cathode having high electron affinity, thus enabling driving at lower voltage. Since more electrons are supplied to the emissive layer and the probability of recombination increases, the luminance efficiency is further improved.

Examples of the heteroaryl ring containing an electron-accepting nitrogen include a triazine ring, a pyridine ring, a pyrazine ring, a pyrimidine ring, a quinoline ring, a quinoxaline ring, a quinazoline ring, a naphthyridine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, a phenanthroimidazole ring and the like.

Examples of the compound having these heteroaryl ring structures include pyridine derivatives, triazine derivatives, quinazoline derivatives, pyrimidine derivatives, benzimidazole derivatives, benzoxazole derivatives, benzthiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives such as bipyridine and terpyridine, quinoxaline derivatives and naphthyridine derivatives. Of these, preferably used derivatives are imidazole derivatives such as tris(N-phenylbenzimidazol-2-yl)benzene, oxadiazole derivatives such as 1,3-bis[(4-tert-butylphenyl)1,3,4-oxadiazolyl]phenylene, triazole derivatives such as N-naphthyl-2,5-diphenyl-1,3,4-triazole, phenanthroline derivatives such as bathocuproine and 1,3-bis(1,10-phenanthrolin-9-yl)benzene, benzoquinoline derivatives such as 2,2'-bis(benzo[h]quinolin-2-yl)-9,9'-spirobifluorene, bipyridine derivatives such as 2,5-bis(6'-(2',2"-bipyridyl))-1,1-dimethyl-3,4-diphenylsilole, terpyridine derivatives such as 1,3-bis(4'-(2,2':6'2"-terpyridinyl))benzene, and naphthyridine derivatives such as bis(1-naphthyl)-4-(1,8-naphthyridin-2-yl)phenylphosphine oxide, from the viewpoint of the electron transportation capability.

When these derivatives have a fused polycyclic aromatic skeleton, the glass transition temperature is improved and the electron mobility increases, and the driving voltage of the organic EL element can be further reduced, which is preferable. Furthermore, considering further improvement in durability life of the element, ease of synthesis and ease of availability of raw materials, the fused polycyclic aromatic skeleton is more preferably a fluoranthene skeleton, an anthracene skeleton, a pyrene skeleton or a phenanthroline skeleton.

Specific examples of preferred electron transporting material include, but are not particularly limited to, the following examples.

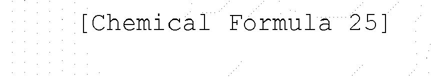

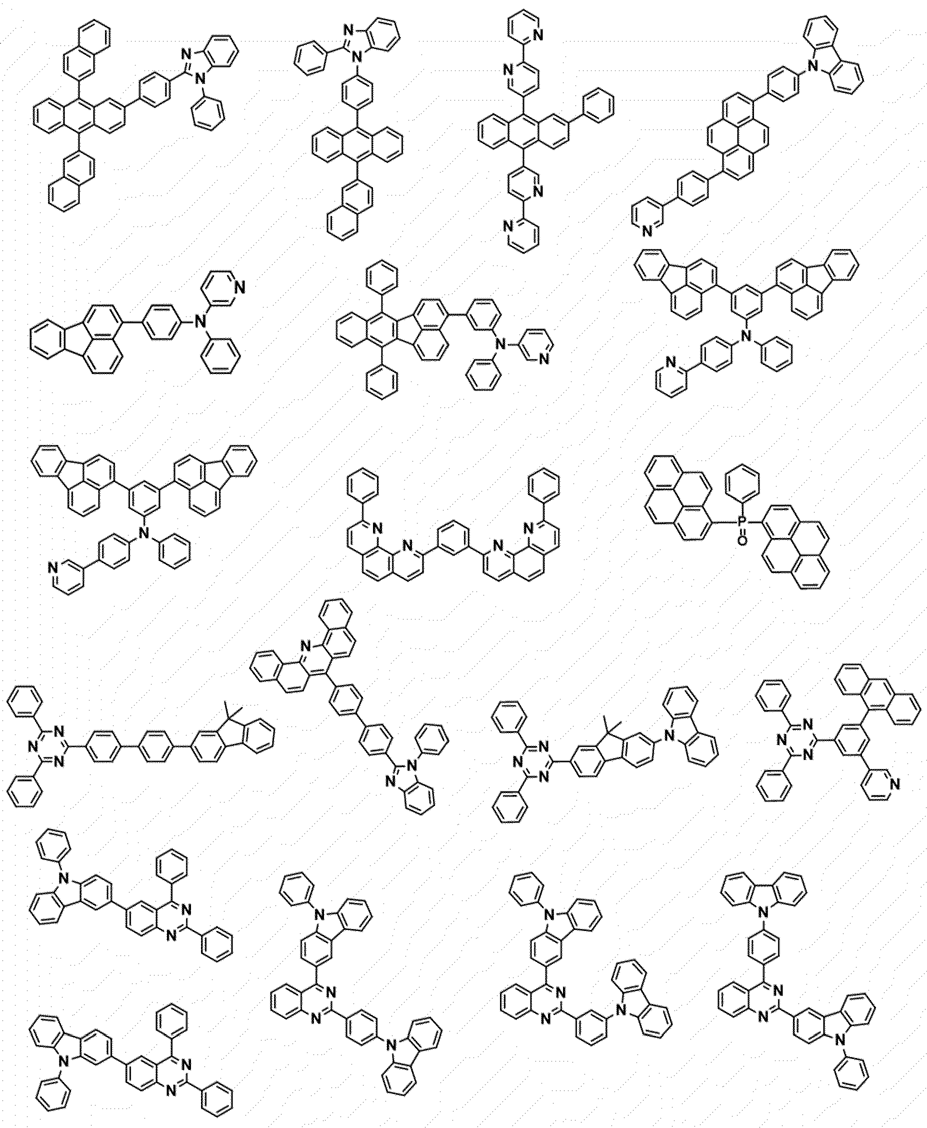

The organic EL element material represented by the general formula (1) is also preferable since it has high electron transportability and exhibits excellent properties as the electron transporting layer.

The electron transporting material may be used alone, or two or more electron transporting materials may be used by mixing, or one or more other electron transporting materials may be mixed used by mixing with the electron transporting material. The electron transporting material may contain a donor compound. As used herein, the donor compound is a compound which facilitates injection of electrons into the electron transporting layer from the cathode or the electron injection layer and also improves electric conductivity of the electron transporting layer by improving the electron injection barrier when used in the electron transporting layer.

Preferred examples of the donor compound include an alkali metal, an inorganic salt containing an alkali metal, a complex of an alkali metal and an organic substance, an alkali earth metal, an inorganic salt containing an alkali earth metal, a complex of an alkali earth metal and an organic substance, rare earth metal and the like. Preferred examples of the alkali metal, alkali earth metal and rare earth metal include alkali metals such as lithium, sodium, potassium, rubidium and cesium, alkali earth metals such as magnesium, calcium, cerium and barium, and rare earth metals such as samarium, europium and ytterbium, which have low work function and are highly effective in improving electron transportation capability. Preferred examples of the alkali metals or alkali earth metal include lithium and cesium from the viewpoint that the driving voltage can be further reduced. A plurality of these metals may be used, and an alloy of these metals may also be used.

In view of easy deposition in vacuum and excellent handling, these metals are preferably in the form of an inorganic salt or a complex with an organic substance rather than an elemental metal. In view of facilitating handling in the the atmospheric air and control of the concentration to be added, these metals are preferably in the form a complex with an organic substance. Examples of the inorganic salts include oxides such as LiO and Li₂O; nitrides; fluorides such as LiF, NaF, and KF; and carbonates such as Li₂CO₃, Na₂CO₃, K₂CO₃, Rb₂CO₃, and Cs₂CO₃. Preferred examples of the organic substance in complexes with an organic substance include quinolinol, benzoquinolinol, pyridylphenol, flavonol, hydroxyimidazopyridine, hydroxybenzoazole, hydroxytriazole and the like. Of these, an alkali metal complex compound, which is a complex of an alkali metal and an organic substance, is preferable from the viewpoint that the driving voltage of the organic EL element can be further reduced. Furthermore, from the viewpoint of ease of synthesis and thermal stability, a complex of lithium and an organic substance is more preferable, and lithium quinolinol (Liq), which is available at relatively low cost, is particularly preferable.

The ionization potential of the electron transporting layer is not particularly limited, but is preferably 5.6 eV or more and 8.0 eV or less, and more preferably 5.6 eV or more and 7.0 eV or less.

### (Electron Injection Layer)

In the present invention, an electron injection layer may be provided between the cathode and the electron transporting layer. The electron injection layer is generally inserted for the purpose of assisting the injection of electrons from the cathode into the electron transporting layer. For insertion, a compound having a heteroaryl ring structure containing an electron-accepting nitrogen may be used, and a layer containing the above donor material may also be used.

Inorganic substances such as an insulator and a semiconductor can also be used for the electron injection layer. By using these materials, the short circuit of the organic EL element can be suppressed to improve the electron injection properties.

Such insulator is preferably at least one metal compound selected from the group consisting of alkali metal chalcogenides, alkali earth metal chalcogenides, alkali metal halides and alkali earth metal halides.

Specifically, examples of preferred alkali metal chalcogenide include Li₂O, Na₂S and Na₂Se. Examples of preferred alkali earth metal chalcogenide include CaO, BaO, SrO, BeO, BaS and CaSe. Examples of preferred alkali metal halide include LiF, NaF, KF, LiCl, KCl and NaCl. Examples of preferred alkali earth metal halide include fluorides such as CaF₂, BaF₂, SrF₂, MgF₂ and BeF₂, and halides other than fluorides.

Furthermore, a complex of an organic substance and metal are also preferably used. When the complex of an organic substance and metal is used for the electron injection layer, the film thickness can be easily adjusted. Preferred examples of the organic substance in an organometallic complex include quinolinol, benzoquinolinol, pyridylphenol, flavonol, hydroxyimidazopyridine, hydroxybenzazole, hydroxytriazole and the like.

The layer containing the organic EL element material represented by the general formula (1) has high electron injection properties and exhibits excellent properties as the electron injection layer, which is preferable. Furthermore, when the organic EL element material represented by the general formula (1) is used for the electron injection layer, it is preferable to dope with the alkali metal or rare earth metal, thus enabling further reduction in driving voltage and further improvement in durability life.

### (Cathode)

The material used for the cathode is not particularly limited as long as it can efficiently inject electrons into the emissive layer. Examples of materials used for the cathode include metals such as platinum, gold, silver, copper, iron, tin, aluminum and indium, or alloys or multilayer laminates of these metals with metals having low work function, such as lithium, sodium, potassium, calcium and magnesium. Of these, metal selected from aluminum, silver and magnesium is preferable in view of the electric resistance value, ease of film formation, stability of a film, luminance efficiency and the like, and it is more preferable that the cathode is composed of magnesium and silver since it is easy to inject electrons into the electron transporting layer and electron injection layer.

### (Protective Layer)

It is preferable to laminate a protective layer (capping layer) on a cathode to protect the cathode. Examples of the material constituting the protective layer (capping material) include, but are not particularly limited to, metals such as platinum, gold, silver, copper, iron, tin, aluminum and indium; alloys using these metals; inorganic substances such as silica, titania and silicon nitride; and organic polymer compounds such as polyvinyl alcohol, polyvinyl chloride and hydrocarbon-based polymer compound. The organic EL element material represented by the general formula (1) can also be utilized as the capping material. However, when the organic EL element has a structure for taking out light from the cathode side (top emission structure), the capping material having light permeability in a visible light region.

### (Charge Generation Layer)

The charge generation layer in the present invention generally comprises a double layer and, specifically, it is preferable to use a p-n junction charge generation layer comprising an n-type charge generation layer and a p-type charge generation layer. The p-n junction charge generation layer generates charges or separates the charges into holes and electrons when a voltage is applied in the organic EL element, thus injecting these holes and electrons into the emissive layer via the hole transporting layer and electron transporting layer. Specifically, the charge generation layer functions as an intermediate charge generation layer between the plurality of emissive layers in the organic EL element in which a plurality of emissive layers are laminated. The n-type charge generation layer supplies electrons to a first emissive layer existing on the anode side, and the p-type charge generation layer supplies holes to a second emissive layer existing on the cathode side. Therefore, it is possible to further improve the luminance efficiency of the organic EL element in which a plurality of emissive layers are laminated and to reduce the driving voltage, and further improve the durability life of the element.

The n-type charge generation layer comprises an n-type dopant material and a host material, and conventional materials can be used as these materials. For example, alkali metals, alkali earth metals or rare earth metals can be used as the n-type dopant material. Preferred n-type dopant material is lithium or ytterbium. A plurality of these metals may be used in combination. Alloys of alkali metals, alkali earth metals or rare earth metals with other metals may also be used. Examples of the metal which can be used as the material for the alloy include, but are not limited to, zinc, cadmium or bismuth. It is possible to use, as the host material, a compound having nitrogen-containing aromatic heterocycles such as phenanthroline derivatives and oligopyridine derivatives. A compound having a phosphine oxide group can also be used. In particular, the organic EL element material or phenanthroline dimer represented by the general formula (1) is preferable since it exhibits excellent properties as a host of the n-type charge generation layer. A plurality of them may be used in combination.

The p-type charge generation layer comprises a p-type dopant material and a host material, and conventional materials can be used as these materials. For example, it is possible to use, as the p-type dopant material, tetrafluoro-7,7,8,8-tetracyanoquinodimethane (F4-TCNQ), tetracyanoquinodimethane derivatives, radialene derivatives, iodine, FeCl₃, FeF₃, SbCl₅ and the like. Radialene derivatives are preferable as the p-type dopant material. Arylamine derivatives are preferable as the host material.

Examples of the method for forming each layer constituting the organic EL element include, but are not limited to, a resistance heating evaporation method, an electron beam evaporation method, a sputtering method, a molecular stacking method, a coating method and the like. In view of element characteristics, a resistance heating evaporation method or an electron beam evaporation method is usually preferable.

Although the total thickness of the organic layer existing between the anode and the cathode varies depending on the resistance value of an emissive substance and cannot be limited, it is preferably 1 to 1,000 nm. The thickness of each of the emissive layer, the electron transporting layer and the hole transporting layer is preferably 1 nm or more and 200 nm or less, and more preferably 5 nm or more and 100 nm or less.

The organic EL element according to the embodiment of the present invention has a function of converting electric energy into light. While a direct current is mainly used as the electric energy, a pulse current or an alternating current can also be used. The values of the electric current and the voltage are not particularly limited. However, considering the power consumption and the life of the element, the values should be selected such that a maximum luminance can be obtained at energy as low as possible.

The organic EL element according to the embodiment of the present invention is preferably used as a display device such as a display which displays in a matrix and/or segment system.

The organic EL element according to the embodiment of the present invention can also be preferably employed as a backlight of various instruments. The backlight is mainly used for the purpose of improving visibility of a display device such as display which itself emits no light, and it is used in liquid crystal displays, watches, audio devices, automobile panels, display plates and signs. The organic EL element of the present invention is preferably used as the backlight of a liquid crystal display, particularly a personal computer, the thickness reduction of which is being studied, and can provide a backlight that is smaller in thickness and weight than conventional products.

The organic EL element according to the embodiment of the present invention is also preferably used as various illumination devices. The organic EL element according to the embodiment of the present invention can achieve both high luminance efficiency and high color purity, and also can be made thinner and lighter, and thus it is possible to realize an illumination device with low power consumption, vivid color and high design quality.

### [Examples]

The present invention will be described by way of Examples, but the present invention is not limited to these Examples.

### Synthesis Example 1: Synthesis of Compound 5

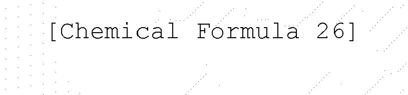

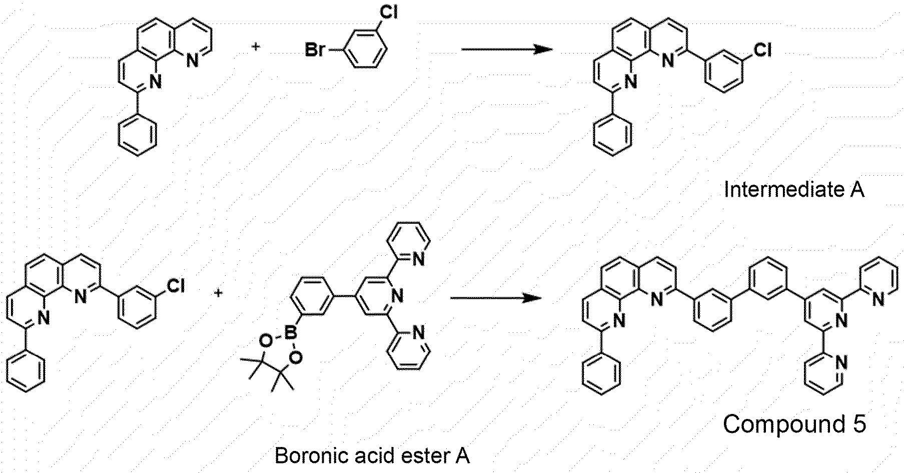

To a mixed solution of 4.0 g of 1-bromo-3-chlorobenzene and 30 ml of tetrahydrofuran, 13 ml of n-butyl lithium (1.6 M hexane solution) was added dropwise under a nitrogen gas flow at 0°C. After stirring at 0°C for 1 hour, the mixture was added dropwise at 0°C to a mixed solution of 4.5 g of 2-phenyl-1,10-phenanthroline and 30 ml of tetrahydrofuran. After temperature rising to room temperature, the reaction solution was extracted with dichloromethane and the solvent was evaporated leaving 100 ml. To the solution thus obtained, 10.0 g of manganese dioxide was added, and after stirring at room temperature for 4 hours, magnesium sulfate was added, followed by filtration and further removal of the solvent by evaporation. The solid thus obtained was purified by silica gel column chromatography, and the solid obtained by removing the solvent by evaporation was vacuum-dried to obtain 6.0 g of an intermediate A.

Subsequently, a mixed solution of 3.0 g of the intermediate A, 3.7 g of a boronic acid ester A, 160 mg of dichlorobis(triphenylphosphinepalladium)dichloride, 7 ml of an aqueous 1.5 M tripotassium phosphate solution and 80 ml of 1,4-dioxane was heated and stirred under reflux under a nitrogen gas flow for 7 hours. After cooling to room temperature, water was added and the precipitated solid was collected by filtration, washed with methanol and then vacuum-dried. After the solid thus obtained was dissolved in a mixed solvent of toluene and pyridine, and then the catalyst was removed using activated carbon. The solid obtained by removing the solvent by evaporation was washed with toluene and methanol, and then vacuum-dried to obtain 3.0 g of a compound 5.

The compound 5 thus obtained was purified by sublimation at about 360°C under a pressure of 1 × 10⁻³ Pa using an oil diffusion pump. The HPLC purity (area % at a measurement wavelength of 254 nm) of the compound 5 before and after sublimation purification was both 99.9%.

After sublimation purification, the structure of the compound 5 was identified by mass spectrum (MS) analysis and ¹H-NMR analysis. The analytical results are shown below. MS(m/z): 640[M+H]⁺
¹H-NMR (400MHz, CDCl₃) δ: 8.94(s, 1H), 8.81-8.87(m, 2H), 8.65-8.74(m, 4H), 8.40-8.52(m, 3H), 8.23-8.40(m, 4H), 7.78-7.99(m, 6H), 7.64-7.78(m, 2H), 7.31-7.50(m, 4H).

### Synthesis Example 2: Synthesis of Compound 10

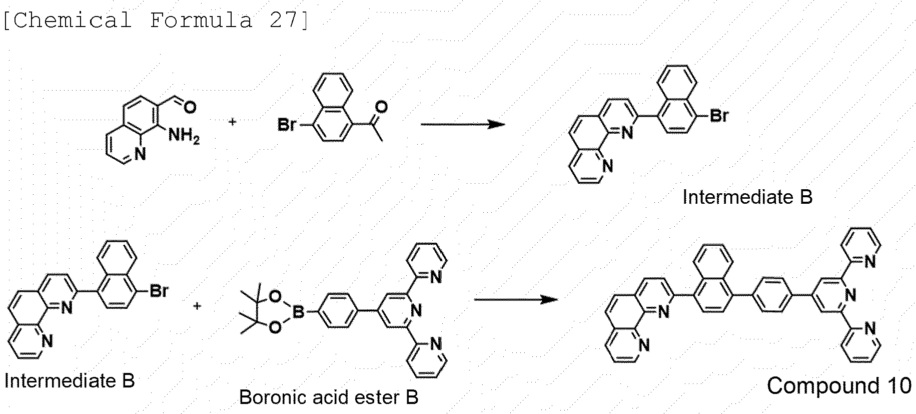

A mixed solution of 10.0 g of 8-aminoquinoline-7-carbaldehyde, 13.1 g of 1-(4-bromonaphthalen-1-yl)ethan-1-one, 50 ml of an aqueous 2.0 M potassium hydroxide solution and 250 ml of ethanol was heated and stirred at 70°C under reflux under a nitrogen gas flow for 5 hours. After cooling to room temperature, water was added and the precipitated solid was collected by filtration, washed with methanol and then vacuum-dried to obtain 12.1 g of an intermediate B.

Subsequently, a mixed solution of 3.0 g of the intermediate B, 3.6 g of a boronic acid ester B, 160 mg of dichlorobis(triphenylphosphinepalladium)dichloride, 7 ml of an aqueous 1.5 M tripotassium phosphate solution and 70 ml of 1,4-dioxane was heated and stirred under reflux under a nitrogen gas flow for 7 hours. After cooling to room temperature, water was added and the precipitated solid was collected by filtration, washed with methanol and then vacuum-dried. After the solid thus obtained was dissolved in a mixed solvent of toluene and pyridine, and then the catalyst was removed using activated carbon. The solid obtained by removing the solvent by evaporation was washed with toluene and methanol, and then vacuum-dried to obtain 2.7 g of a compound 10.

The compound 10 thus obtained was purified by sublimation at about 360°C under a pressure of 1 × 10⁻³ Pa using an oil diffusion pump. The HPLC purity (area % at a measurement wavelength of 254 nm) of the compound 1 before and after sublimation purification was both 99.9%.

After sublimation purification, the structure of the compound 10 was identified by mass spectrum (MS) analysis and ¹H-NMR analysis. The analytical results are shown below. MS(m/z): 614[M+H]⁺
¹H-NMR (400MHz, CDCl₃) δ: 9.23(s, 1H), 8.84-8.88(m, 2H), 8.65-8.79(m, 3H), 8.34-8.43(m, 1H), 8.24-8.34(m, 1H), 7.82-8.20(m, 9H), 7.58-7.74(m, 4H), 7.42-7.52(m, 2H), 7.31-7.42(m, 2H).

Subsequently, the evaluation method in each Example will be described.

### (Driving Voltage)

Each of the elements obtained in Examples 1 to 12 and Comparative Examples 1 to 14 was direct-current driven at 10 mA/cm² and the initial driving voltage was measured. Furthermore, the voltage was measured when direct-current driving was performed at a current density of 10 mA/cm² in an environment at a temperature of 70°C for 100 hours, and the amount of increase in voltage from the initial driving voltage was calculated.

Each of the organic EL elements obtained in Examples 13 to 24 and Comparative Examples 15 to 28 was lit on at a luminance of 1,000 cd/m², and the initial driving voltage was measured. The voltage was measured when constant-current driving was performed at room temperature at a current density of 10 mA/cm² for 100 hours. The amount of increase in voltage was calculated after 100 hours from the start of the measurement.

Each of the organic EL elements obtained in Examples 25 to 63 and Comparative Examples 29 to 70 was driven at a current density of 10 mA/cm², and the initial driving voltage was measured.

The lower the initial driving voltage, the lower the voltage required to drive, so that it is possible to evaluate that the luminous efficiency (luminance/power) is excellent. It is possible to evaluate that the smaller the amount of increase in voltage, the more the durability life is excellent.

### (External Quantum Efficiency)

Each of the organic EL elements obtained in Examples 13 to 24 and Comparative Examples 15 to 28 was lit on at a current density of 10 mA/cm², and the external quantum efficiency was measured and then the luminance efficiency was evaluated. It is possible to evaluate that the higher the external quantum efficiency, the more the luminance efficiency is excellent.

### (Luminance)

Each of the organic EL elements obtained in Examples 25 to 63 and Comparative Examples 29 to 70 was lit on at 10 mA/cm², and the luminance was measured and then the luminance efficiency was evaluated. It is possible to evaluate that the higher the luminance, the more the luminance efficiency is excellent.

### (Durability Life)

Each of the organic EL elements obtained in Examples 13 to 63 and Comparative Examples 15 to 70 was continuously driven at a constant current of 10 mA/cm². The time for luminance to decrease by 20% from the luminance at the start of the measurement was measured and defined as the endurance life.

### Example 1

A glass substrate (manufactured by Geomatec Co., Ltd., 11 Ω/□, sputtered product) on which an ITO transparent conductive film of 125 nm was deposited as an anode was cut into a size of 38 mm × 46 mm, followed by etching. The substrate thus obtained was ultrasonically cleaned for 15 minutes using "Semico Clean" (registered trademark) 56 (trade name, manufactured by Furuuchi Chemical Co., Ltd.) and then cleaned with ultrapure water. This substrate was treated with UV-ozone for 1 hour immediately before fabrication of the element and placed in a vacuum deposition apparatus, and then evacuated to the degree of vacuum of 5 × 10⁻⁴ Pa or less. By a resistance heating method, the compound 1 and the dopant metal element Li were deposited in a thickness of 100 nm at a vapor deposition rate ratio of the compound 1 and Li of 99:1 to form a layer with a weight ratio of 99:1. Thereafter, aluminum was deposited in a thickness of 60 nm to form a cathode, thus fabricating an element of 5 mm × 5 mm square. The film thickness as used herein is a crystal oscillation type film thickness monitor display value, and is common to other Examples and Comparative Examples.

This element was evaluated by the method mentioned above and, as a result, the initial driving voltage was 0.029 V and the amount of increase in voltage after driving at 70°C for 100 hours was 0.001 V.

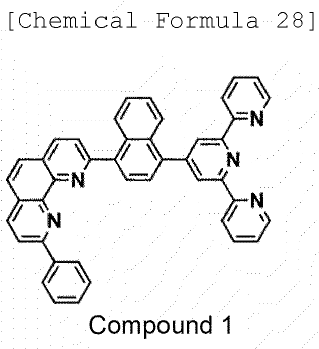

### Examples 2 to 6, Reference Examples 7 to 12, Comparative Examples 1 to 14

In the same manner as in Example 1, expect that the compounds used and the vapor deposition rate ratio of the compound and the metal element was changed as shown in Table 1, elements were fabricated. The results of the respective Example, Reference Examples, and Comparative Examples are shown in Table 1. Compounds 2 to 26 are compounds shown below.

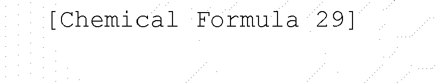

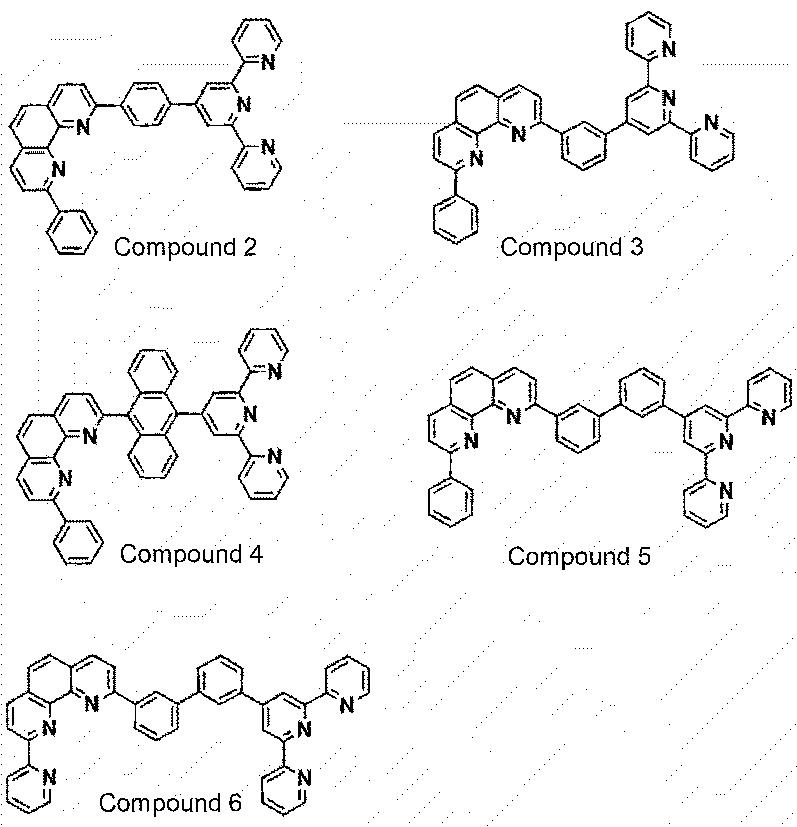

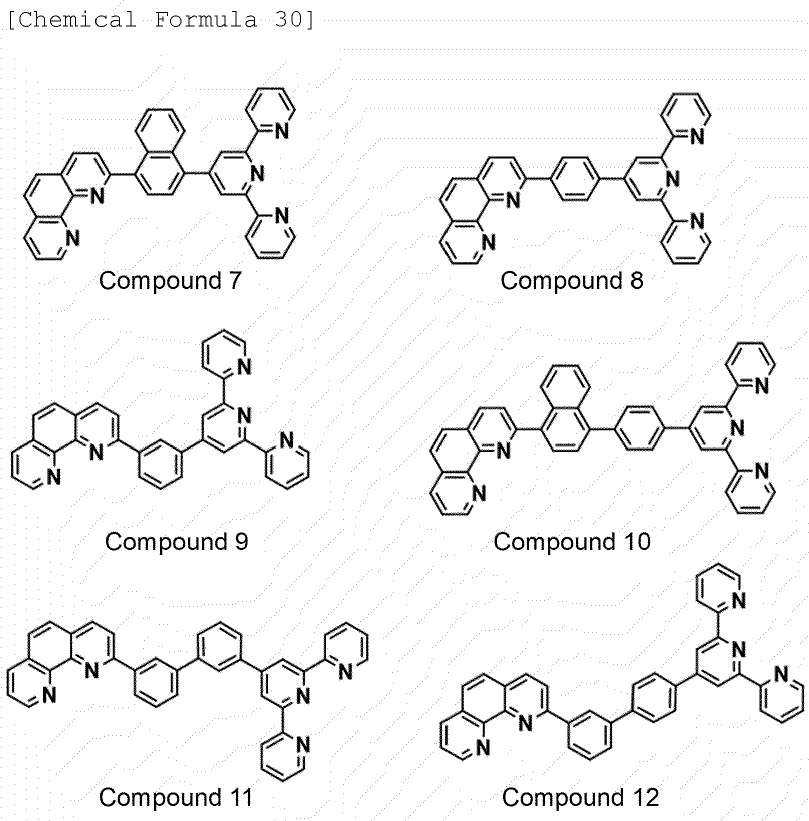

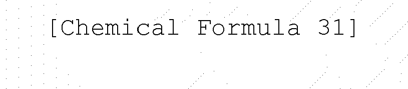

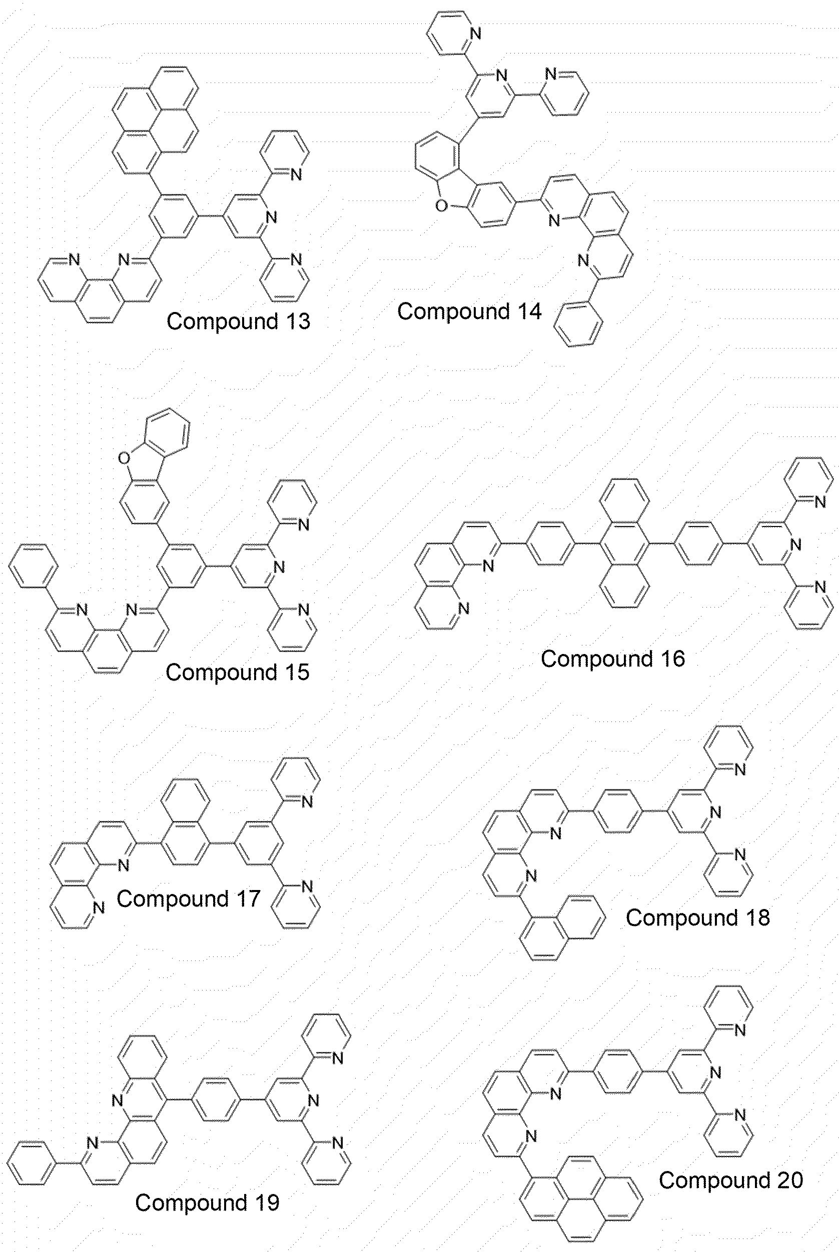

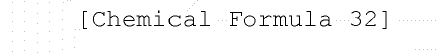

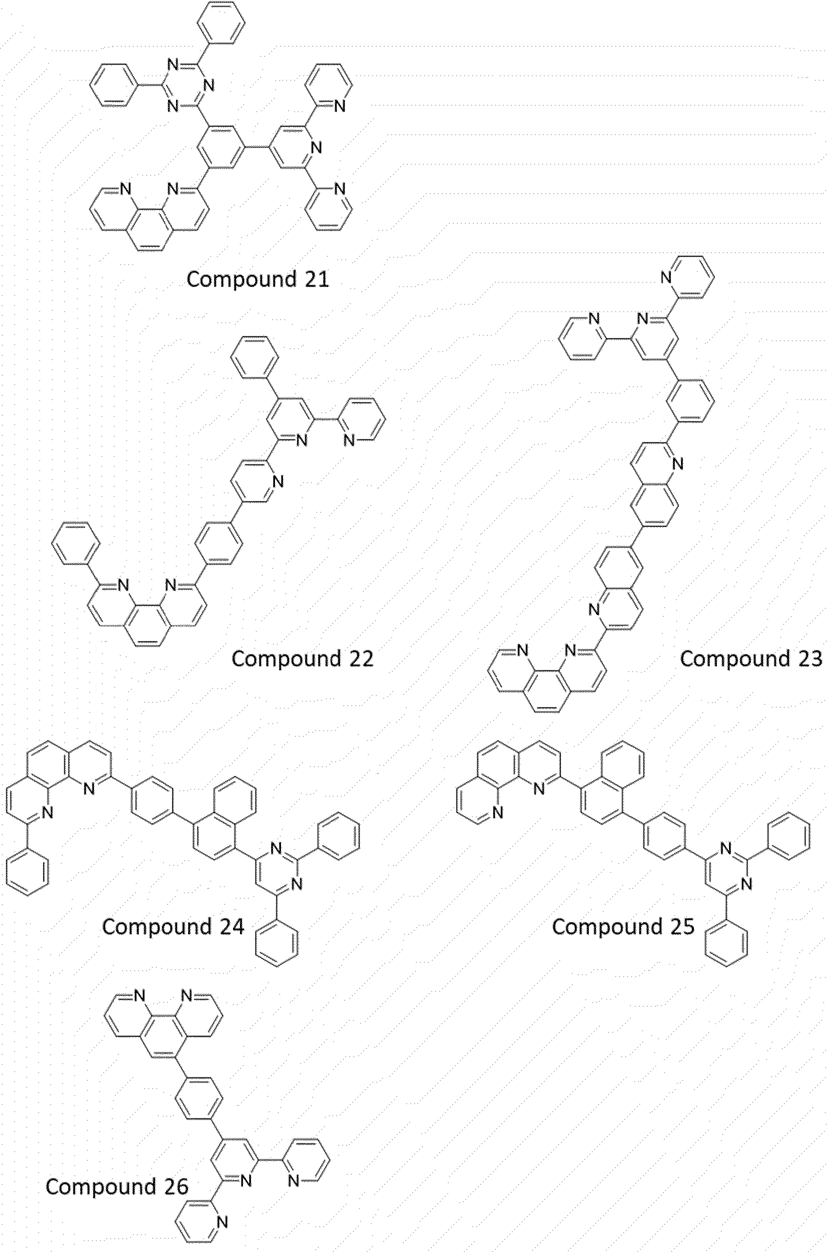

**[Table 1]**

| | Compound | Deposition rate ratio of compound and metal element | Initial driving voltage (V) | Durability life |
|---|---|---|---|---|
| | | | | Amount of increase in voltage after driving at 70°C for 100 hours (V) |
| Example 1 | Compound 1 | 99:1 | 0.029 | 0.001 |
| Example 2 | Compound 2 | 99:1 | 0.031 | 0.001 |
| Example 3 | Compound 3 | 99:1 | 0.031 | 0.001 |
| Example 4 | Compound 4 | 99:1 | 0.039 | 0.005 |
| Example 5 | Compound 5 | 99:1 | 0.052 | 0.011 |
| Example 6 | Compound 6 | 99:1 | 0.071 | 0.013 |
| Reference Example 7 | Compound 7 | 99:1 | 0.029 | 0.002 |
| Reference Example 8 | Compound 8 | 99:1 | 0.032 | 0.003 |
| Reference Example 9 | Compound 9 | 99:1 | 0.031 | 0.003 |
| Reference Example 10 | Compound 10 | 99:1 | 0.034 | 0.004 |
| Reference Example 11 | Compound 11 | 99:1 | 0.039 | 0.010 |
| Reference Example 12 | Compound 12 | 99:1 | 0.040 | 0.011 |
| Comparative Example 1 | Compound 13 | 99:1 | 0.141 | 0.101 |
| Comparative Example 2 | Compound 14 | 99:1 | 0.101 | 0.126 |
| Comparative Example 3 | Compound 15 | 99:1 | 0.113 | 0.134 |
| Comparative Example 4 | Compound 16 | 99:1 | 0.121 | 0.154 |
| Comparative Example 5 | Compound 17 | 99:1 | 0.201 | 0.200 |
| Comparative Example 6 | Compound 18 | 99:1 | 0.100 | 0.085 |
| Comparative Example 7 | Compound 19 | 99:1 | 0.131 | 0.092 |
| Comparative Example 8 | Compound 20 | 99:1 | 0.152 | 0.091 |
| Comparative Example 9 | Compound 21 | 99:1 | 0.121 | 0.112 |
| Comparative Example 10 | Compound 22 | 99:1 | 0.134 | 0.095 |
| Comparative Example 11 | Compound 23 | 99:1 | 0.117 | 0.098 |
| Comparative Example 12 | Compound 24 | 99:1 | 0.108 | 0.135 |
| Comparative Example 13 | Compound 25 | 99:1 | 0.110 | 0.120 |
| Comparative Example 14 | Compound 26 | 99:1 | 0.123 | 0.131 |

### Example 13

A glass substrate (manufactured by Geomatec Co., Ltd., 11 Ω/□, sputtered product) on which an ITO transparent conductive film of 165 nm was deposited as an anode was cut into a size of 38 mm × 46 mm, followed by etching. The substrate thus obtained was ultrasonically cleaned for 15 minutes using "Semico Clean" (registered trademark) 56 (trade name, manufactured by Furuuchi Chemical Co., Ltd.) and then cleaned with ultrapure water. This substrate was treated with UV-ozone for 1 hour immediately before fabrication of the element and placed in a vacuum deposition apparatus, and then evacuated to the degree of vacuum of 5 × 10⁻⁴ Pa or less. By a resistance heating method, first, HAT-CN₆ as a material of a hole injection layer was deposited in a thickness of 5 nm and HT-1 as a material of a hole transporting layer was deposited in a thickness of 50 nm. Then, a mixed layer of a host material H-1 and a dopant material D-1 as an emissive layer was deposited in a thickness of 20 nm so that the doping concentration was 5% by weight. Then, ET-1 and 2E-1 were deposited in a thickness of 35 nm as an electron transporting layer so that the vapor deposition rate ratio of ET-1 and 2E-1 was 1:1. Then, a compound 1 and a metal element Li as a dopant were deposited in a thickness of 10 nm as an electron injection layer so that the vapor deposition rate ratio of the compound 1 and Li was 99:1. Thereafter, aluminum was deposited in a thickness of 60 nm to form a cathode, thus fabricating an organic EL element of 5 mm × 5 mm square.

When this organic EL element was evaluated by the method mentioned above and, as a result, the initial driving voltage was 4.00 V, the external quantum efficiency (luminance efficiency) was 5.79%, the durability life was 1,080 hours, and the amount of increase in voltage was 0.001 V when driven at room temperature for 100 hours. HAT-CN₆, HT-1, H-1, D-1, ET-1 and 2E-1 are compounds shown below.

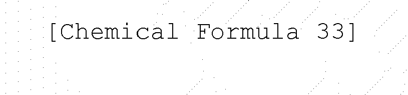

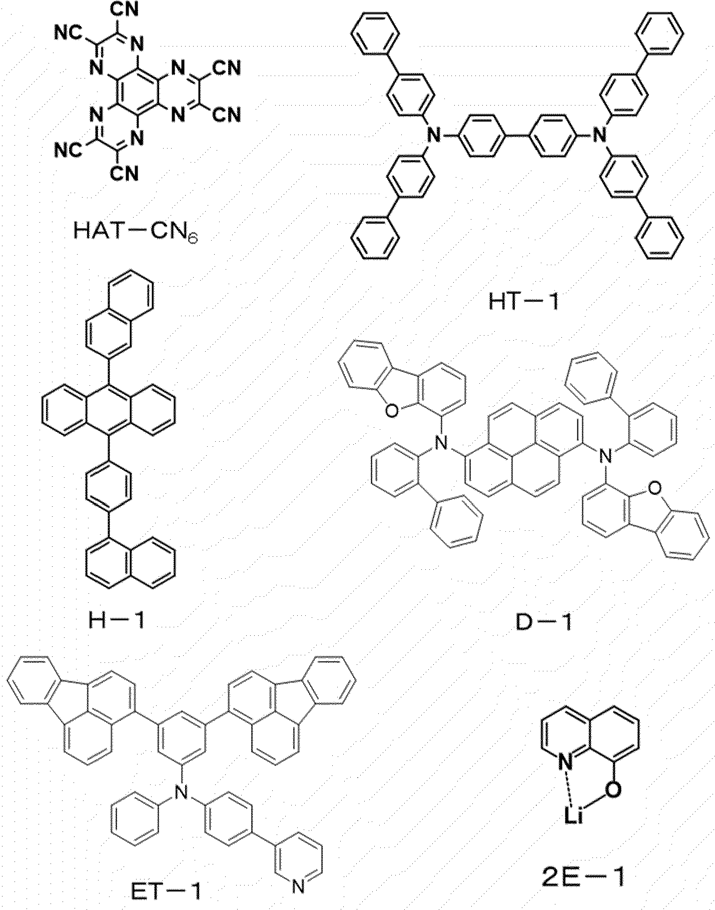

### Examples 14 to 18, Reference Examples 19 to 24, Comparative Examples 15 to 28

In the same manner as in Example 13, expect that the compounds used and the vapor deposition rate ratio of the compound and the metal element was changed as shown in Table 2, organic EL elements were fabricated. The results of the respective Example, Reference Examples, and Comparative Examples are shown in Table 2.

**[Table 2]**

| | Electron injection layer | | Luminance efficiency | | Durability life | |
|---|---|---|---|---|---|---|
| | Compound | Deposition rate ratio of compound and metal element | Initial driving voltage (V) | External quantum efficiency (%) | Time for luminance to decrease by 20% from initial luminance (h) | Amount of increase in voltage after driving for 100 hours (V) |
| Example 13 | Compound 1 | 99:1 | 4.00 | 5.79 | 1,080 | 0.001 |
| Example 14 | Compound 2 | 99:1 | 4.05 | 5.70 | 1,070 | 0.001 |
| Example 15 | Compound 3 | 99:1 | 4.05 | 5.61 | 1,060 | 0.002 |
| Example 16 | Compound 4 | 99:1 | 4.21 | 5.48 | 1,040 | 0.007 |
| Example 17 | Compound 5 | 99:1 | 4.46 | 5.31 | 1,030 | 0.012 |
| Example 18 | Compound 6 | 99:1 | 4.43 | 5.22 | 950 | 0.014 |
| Reference Example 19 | Compound 7 | 99:1 | 4.01 | 5.73 | 1,000 | 0.003 |
| Reference Example 20 | Compound 8 | 99:1 | 4.11 | 5.68 | 980 | 0.004 |
| Reference Example 21 | Compound 9 | 99:1 | 4.12 | 5.68 | 970 | 0.006 |
| Reference Example 22 | Compound 10 | 99:1 | 4.19 | 5.43 | 960 | 0.008 |
| Reference Example 23 | Compound 11 | 99:1 | 4.21 | 5.31 | 950 | 0.013 |
| Reference Example 24 | Compound 12 | 99:1 | 4.51 | 5.20 | 920 | 0.015 |
| Comparative Example 15 | compound 13 | 99:1 | 5.64 | 4.71 | 500 | 0.121 |
| Comparative Example 16 | Compound 14 | 99:1 | 5.58 | 4.50 | 520 | 0.151 |
| Comparative Example 17 | Compound 15 | 99:1 | 5.60 | 4.49 | 470 | 0.142 |
| Comparative Example 18 | Compound 16 | 99:1 | 5.62 | 4.38 | 420 | 0.178 |
| Comparative Example 19 | Compound 17 | 99:1 | 6.10 | 4.41 | 480 | 0.200 |
| Comparative Example 20 | Compound 18 | 99:1 | 5.57 | 4.69 | 510 | 0.100 |
| Comparative Example 21 | Compound 19 | 99:1 | 5.63 | 4.31 | 490 | 0.132 |
| Comparative Example 22 | Compound 20 | 99:1 | 5.61 | 4.65 | 480 | 0.161 |
| Comparative Example 23 | Compound 21 | 99:1 | 5.43 | 4.28 | 420 | 0.130 |
| Comparative Example 24 | Compound 22 | 99:1 | 5.50 | 4.15 | 500 | 0.121 |
| Comparative Example 25 | Compound 23 | 99:1 | 5.31 | 4.20 | 480 | 0.135 |
| Comparative Example 26 | Compound 24 | 99:1 | 5.42 | 4.46 | 470 | 0.150 |
| Comparative Example 27 | Compound 25 | 99:1 | 5.40 | 4.38 | 510 | 0.134 |
| Comparative Example 28 | Compound 26 | 99:1 | 5.53 | 4.40 | 490 | 0.144 |

### Example 25

A glass substrate (manufactured by Geomatec Co., Ltd., 11 Ω/□, sputtered product) on which an ITO transparent conductive film of 165 nm was deposited as an anode was cut into a size of 38 mm × 46 mm, followed by etching. The substrate thus obtained was ultrasonically cleaned for 15 minutes using "Semico Clean" (registered trademark) 56 (trade name, manufactured by Furuuchi Chemical Co., Ltd.) and then cleaned with ultrapure water. This substrate was treated with UV-ozone for 1 hour immediately before fabrication of the element and placed in a vacuum deposition apparatus, and then evacuated to the degree of vacuum of 5 × 10⁻⁴ Pa or less. By a resistance heating method, first, HAT-CN₆ as a material of a hole injection layer was deposited in a thickness of 5 nm. Then, a light emitting unit (first lightemitting unit) comprising a hole transporting layer, an emissive layer and an electron transporting layer was formed on the hole injection layer.

Specifically, HT-1 as a material of a hole transporting layer was deposited in a thickness of 50 nm, and then a mixed layer of a host material H-1 and a dopant material D-1 as an emissive layer was deposited in a thickness of 20 nm so that the doping concentration became 5% by weight. Then, ET-1 and 2E-1 as materials of an electron transporting layer were deposited in a thickness of 35 nm at a vapor deposition rate ratio of ET-1 and 2E-1 of 1:1.

On the first light emitting unit, a compound 1 and a metal element Li as a dopant as materials of an n-type charge generation layer were deposited in a thickness of 10 nm so that the vapor deposition rate ratio of the compound 1 and Li was 99:1, and then HAT-CN₆ as a material of was a p-type charge generation layer was deposited in a thickness of 10 nm.

Following the charge generation layer, a second light emitting unit was formed in the same manner as in the first light emitting unit. Thereafter, a compound 1 and a dopant metal element Li as materials of an electron injection layer was deposited in a thickness of 10 nm so that the vapor deposition rate ratio of the compound 1 and Li was 99:1, and then aluminum was deposited in a thickness of 60 nm to form a cathode, thus fabricating an organic EL element of 5 mm × 5 mm square.

This organic EL element was evaluated by the method mentioned above and, as a result, the initial driving voltage was 8.21 V, the luminance was 1,760 cd/m², and the endurance life was 2,620 hours.

### Examples 26 to 30, 37, and 38, Reference Examples 31 to 36, Comparative Examples 29 to 42

In the same manner as in Example 25, expect that the compounds used and the vapor deposition rate ratio of the compound and the metal element was changed as shown in Table 3, organic EL elements were fabricated. In Example 37, a compound 1 and an ET-2 metal element Li as materials of an n-type charge generation layer were deposited in a thickness of 10 nm so that the deposition rate ratio of the compound 1, ET-2 and Li was 49.5:49.5:1. In Example 38, a mixed layer of a host material H-1 and a dopant material D-2 as an emissive layer was deposited in a thickness of 20 nm so that the doping concentration was 5% by weight. The results of the respective Examples, Reference Examples, and Comparative Examples are shown in Table 3. D-2 and ET-2 are compounds shown below.

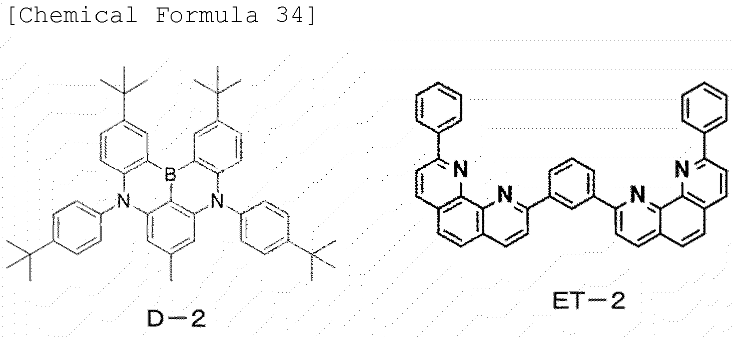

**[Table 3]**

| | Emissive layer | n-Type charge generation layer | | Luminance efficiency | | Durability life |
|---|---|---|---|---|---|---|
| | Dopant material | Compound | Deposition rate ratio of compound and metal element | Initial driving voltage (V) | Luminance (cd/m²) | Time for luminance to decrease by 20% from initial luminance (h) |
| Example 25 | D-1 | Compound 1 | 99:1 | 8.21 | 1,760 | 2,620 |
| Example 26 | D-1 | Compound 2 | 99:1 | 8.42 | 1,730 | 2,570 |
| Example 27 | D-1 | Compound 3 | 99:1 | 8.40 | 1,700 | 2,570 |
| Example 28 | D-1 | Compound 4 | 99:1 | 8.58 | 1,670 | 2,580 |
| Example 29 | D-1 | Compound 5 | 99:1 | 8.73 | 1,640 | 2,500 |
| Example 30 | D-1 | Compound 6 | 99:1 | 8.71 | 1,600 | 2,330 |
| Reference Example 31 | D-1 | Compound 7 | 99:1 | 8.20 | 1,750 | 2,500 |
| Reference Example 32 | D-1 | Compound 8 | 99:1 | 8.35 | 1,720 | 2,450 |
| Reference Example 33 | D-1 | Compound 9 | 99:1 | 8.36 | 1,680 | 2,450 |
| Reference Example 34 | D-1 | Compound 10 | 99:1 | 8.64 | 1,620 | 2,320 |
| Reference Example 35 | D-1 | Compound 11 | 99:1 | 8.58 | 1,630 | 2,310 |
| Reference Example 36 | D-1 | Compound 12 | 99:1 | 8.80 | 1,580 | 2,280 |
| Example 37 | D-1 | Compound 1:ET-2 | 99:1 | 8.15 | 1,850 | 2,750 |
| Example 38 | D-2 | Compound 1 | 99:1 | 8.21 | 1,880 | 2,760 |
| Comparative Example 29 | D-1 | Compound 13 | 99:1 | 12.10 | 1,150 | 1,100 |
| Comparative Example 30 | D-1 | Compound 14 | 99:1 | 11.74 | 1,070 | 1,160 |
| Comparative Example 31 | D-1 | Compound 15 | 99:1 | 11.60 | 1,060 | 1000 |
| Comparative Example 32 | D-1 | Compound 16 | 99:1 | 12.00 | 1,000 | 1,020 |
| Comparative Example 33 | D-1 | Compound 17 | 99:1 | 13.80 | 1,030 | 1,070 |
| Comparative Example 34 | D-1 | Compound 18 | 99:1 | 11.77 | 1,100 | 1,150 |
| Comparative Example 35 | D-1 | Compound 19 | 99:1 | 11.65 | 1,010 | 1,140 |
| Comparative Example 36 | D-1 | Compound 20 | 99:1 | 11.66 | 1,130 | 1,110 |
| Comparative Example 37 | D-1 | Compound 21 | 99:1 | 11.42 | 1,000 | 1,050 |
| Comparative Example 38 | D-1 | Compound 22 | 99:1 | 11.20 | 1,080 | 10,00 |
| Comparative Example 39 | D-1 | Compound 23 | 99:1 | 11.45 | 1,000 | 960 |
| Comparative Example 40 | D-1 | Compound 24 | 99:1 | 11.90 | 1,100 | 970 |
| Comparative Example 41 | D-1 | Compound 25 | 99:1 | 11.80 | 1,050 | 1,050 |
| Comparative Example 42 | D-1 | Compound 26 | 99:1 | 11.85 | 1,100 | 1,000 |

### Example 39

In the same manner as in Example 25, expect that a compound 1 was used in place of ET-1 in the formation of the electron transporting layer, and ET-2 was used in place of the compound 1 in the formation of the n-type charge generation layer, an organic EL element was fabricated.

This organic EL element was evaluated by the method mentioned above and, as a result, the initial driving voltage was 8.20 V, the luminance was 1,790 cd/m², and the endurance life was 2,600 hours.

### Examples 40 to 44, Reference Examples 45 to 50, Comparative Examples 43 to 56

In the same manner as in Example 39, expect that the compounds used and the vapor deposition rate ratio of the compound and the metal element were changed as shown in Table 4, organic EL elements were fabricated. The results of the respective Examples, Reference Examples, and Comparative Examples are shown in Table 4.

**[Table 4]**

| | Electron transporting layer | n-Type charge generation layer | Luminance efficiency | | Durability life |
|---|---|---|---|---|---|
| | Compound | Deposition rate ratio of ET-2 and metal element | Initial driving voltage (V) | Luminance (cd/m²) | Time for luminance to decrease by 20% from initial luminance (h) |
| Example 39 | Compound 1 | 99:1 | 8.20 | 1,790 | 2,600 |
| Example 40 | Compound 2 | 99:1 | 8.38 | 1,750 | 2,550 |
| Example 41 | Compound 3 | 99:1 | 8.38 | 1,710 | 2,530 |
| Example 42 | Compound 4 | 99:1 | 8.47 | 1,680 | 2,420 |
| Example 43 | Compound 5 | 99:1 | 8.60 | 1,620 | 2,400 |
| Example 44 | Compound 6 | 99:1 | 8.61 | 1,580 | 2,300 |
| Reference Example 45 | Compound 7 | 99:1 | 8.20 | 1,760 | 2,510 |
| Reference Example 46 | Compound 8 | 99:1 | 8.39 | 1,740 | 2,470 |
| Reference Example 47 | Compound 9 | 99:1 | 8.40 | 1,700 | 2,470 |
| Reference Example 48 | Compound 10 | 99:1 | 8.62 | 1,650 | 2,400 |
| Reference Example 49 | Compound 11 | 99:1 | 8.61 | 1,620 | 2,330 |
| Reference Example 50 | Compound 12 | 99:1 | 8.81 | 1,600 | 2,280 |
| Comparative Example 43 | Compound 13 | 99:1 | 11.80 | 1,150 | 1,130 |
| Comparative Example 44 | Compound 14 | 99:1 | 11.65 | 1,090 | 1,200 |
| Comparative Example 45 | Compound 15 | 99:1 | 11.40 | 1,080 | 1,060 |
| Comparative Example 46 | Compound 16 | 99:1 | 11.90 | 1,030 | 1,080 |
| Comparative Example 47 | Compound 17 | 99:1 | 13.21 | 1,000 | 1,100 |
| Comparative Example 48 | Compound 18 | 99:1 | 11.68 | 1,120 | 1,190 |
| Comparative Example 49 | Compound 19 | 99:1 | 11.59 | 1,000 | 1,200 |
| Comparative Example 50 | Compound 20 | 99:1 | 11.57 | 1,080 | 1150 |
| Comparative Example 51 | Compound 21 | 99:1 | 11.40 | 1,040 | 1120 |
| Comparative Example 52 | Compound 22 | 99:1 | 11.15 | 1,060 | 1,020 |
| Comparative Example 53 | Compound 23 | 99:1 | 11.20 | 1,020 | 1,000 |
| Comparative Example 54 | Compound 24 | 99:1 | 11.85 | 1,080 | 970 |
| Comparative Example 55 | Compound 25 | 99:1 | 11.50 | 1,070 | 1,050 |
| Comparative Example 56 | Compound 26 | 99:1 | 11.85 | 10,90 | 1,020 |

### Examples 51 to 56, and 63, Reference Examples 57 to 62, Comparative Examples 57 to 70

In the same manner as in Example 25, expect that the type of the metal element and the vapor deposition rate ratio were changed as shown in Table 5, organic EL elements were fabricated.

**[Table 5]**

| | n-Type charge generation layer | | | Luminance efficiency | | Durability life |
|---|---|---|---|---|---|---|
| | Metal element | Compound | Deposition rate ratio of compound and metal element | Initial driving voltage (V) | Luminance (cd/m²) | Time for luminance to decrease by 20% from initial luminance (h) |
| Example 51 | Yb | Compound 1 | 95:5 | 8.20 | 1,760 | 2,620 |
| Example 52 | Yb | Compound 2 | 95:5 | 8.31 | 1,750 | 2,600 |
| Example 53 | Yb | Compound 3 | 95:5 | 8.23 | 1,780 | 2,650 |
| Example 54 | Yb | Compound 4 | 95:5 | 8.49 | 1,700 | 2,600 |
| Example 55 | Yb | Compound 5 | 95:5 | 8.52 | 1,680 | 2,520 |
| Example 56 | Yb | Compound 6 | 95:5 | 8.63 | 1,620 | 2,340 |
| Reference Example 57 | Yb | Compound 7 | 95:5 | 8.20 | 1,740 | 2,510 |
| Reference Example 58 | Yb | Compound 8 | 95:5 | 8.28 | 1,730 | 2,480 |
| Reference Example 59 | Yb | Compound 9 | 95:5 | 8.40 | 1,680 | 2,420 |
| Reference Example 60 | Yb | Compound 10 | 95:5 | 8.58 | 1,610 | 2,330 |
| Reference Example 61 | Yb | Compound 11 | 95:5 | 8.61 | 1,620 | 2,310 |
| Reference Example 62 | Yb | Compound 12 | 95:5 | 8.75 | 1,600 | 2,290 |
| Example 63 | Yb | Compound 1:ET-2 | 95:5 | 8.14 | 1,840 | 2,770 |
| Comparative Example 57 | Yb | Compound 13 | 95:5 | 13.20 | 1,000 | 1,050 |
| Comparative Example 58 | Yb | Compound 14 | 95:5 | 12.81 | 960 | 1,100 |
| Comparative Example 59 | Yb | Compound 15 | 95:5 | 12.70 | 1,010 | 1,010 |
| Comparative Example 60 | Yb | Compound 16 | 95:5 | 12.65 | 950 | 970 |
| Comparative Example 61 | Yb | Compound 17 | 95:5 | 14.22 | 800 | 1,020 |
| Comparative Example 62 | Yb | Compound 18 | 95:5 | 12.79 | 1,000 | 1,040 |
| Comparative Example 63 | Yb | Compound 19 | 95:5 | 12.47 | 910 | 1,080 |
| Comparative Example 64 | Yb | Compound 20 | 95:5 | 12.69 | 1,020 | 1,090 |
| Comparative Example 65 | Yb | Compound 21 | 95:5 | 12.51 | 970 | 1,000 |
| Comparative Example 66 | Yb | Compound 22 | 95:5 | 12.10 | 970 | 980 |
| Comparative Example 67 | Yb | Compound 23 | 95:5 | 12.00 | 980 | 950 |
| Comparative Example 68 | Yb | Compound 24 | 95:5 | 12.20 | 1,000 | 970 |
| Comparative Example 69 | Yb | Compound 25 | 95:5 | 12.55 | 1,020 | 1,000 |
| Comparative Example 70 | Yb | Compound 26 | 95:5 | 12.90 | 990 | 1,000 |

## Claims

1. An organic EL element material represented by the following general formula (1): 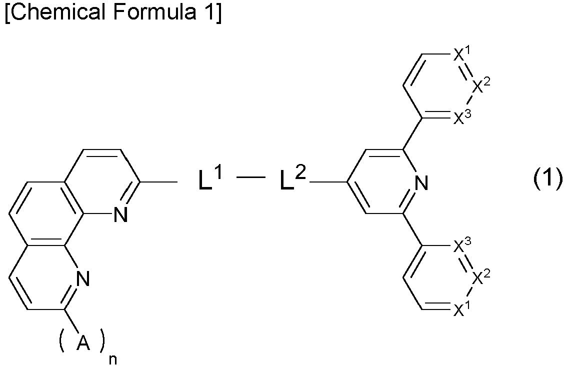 wherein, in the general formula (1), X³ is a nitrogen atom, and X¹ and X² are methine groups; L¹ is a substituted or unsubstituted phenylene group, a substituted or unsubstituted naphthylene group, or a substituted or unsubstituted anthrylene group, and L² is a single bond, a substituted or unsubstituted phenylene group, a substituted or unsubstituted naphthylene group, or a substituted or unsubstituted anthrylene group, in which when these groups are substituted, the substituent is an alkyl group or an alkoxy group; A is a phenyl group or a pyridyl group; and n is 1.

2. The organic EL element material according to claim 1, wherein A is a phenyl group in the general formula (1).

3. The organic EL element material according to claim 1 or 2, wherein L¹ or L² is a naphthylene group in the general formula (1).

4. The organic EL element material according to any one of claims 1 to 3, wherein L² is a single bond in the general formula (1).

5. An organic EL element comprising an anode and a cathode, and at least an electron transporting layer and an emissive layer existing between the anode and the cathode, which is a light emitting element that emits light from electric energy, wherein the electron transporting layer contains the organic EL element material according to any one of claims 1 to 4.

6. The organic EL element according to claim 5, wherein the electron transporting layer further contains an alkali metal complex compound.

7. An organic EL element comprising an anode and a cathode, and at least a charge generation layer and an emissive layer existing between the anode and the cathode, which is a light emitting element that emits light from electric energy, wherein the charge generation layer contains the organic EL element material according to any one of claims 1 to 4.

8. The organic EL element according to claim 7, wherein the charge generation layer further contains a phenanthroline dimer.

9. The organic EL element according to claim 7 or 8, wherein the charge generation layer further contains an alkali metal or a rare earth metal.

10. The organic EL element according to claim 9, wherein the alkali metal is Li.

11. The organic EL element according to claim 9, wherein the rare earth metal is Yb.

12. An organic EL element comprising an anode and a cathode, and at least an electron injection layer and an emissive layer existing between the anode and the cathode, which is a light emitting element that emits light from electric energy, wherein the electron injection layer contains the compound according to any one of claims 1 to 4.

13. The organic EL element according to any one of claims 5 to 12, wherein the emissive layer contains a compound represented by the following general formula (2): 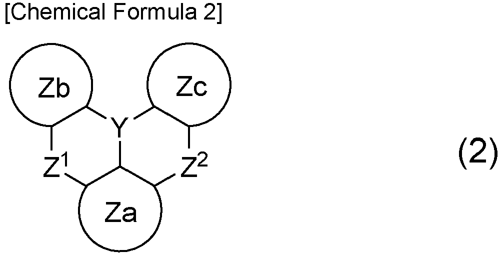 wherein, in the general formula (2), a Za ring, a Zb ring and a Zc ring are each independently a substituted or unsubstituted aryl ring having 6 to 30 ring-forming carbon atoms, or a substituted or unsubstituted heteroaryl ring having 5 to 30 ring-forming atoms; Z¹ and Z² are each independently an oxygen atom, NRa (a nitrogen atom having a substituent Ra) or a sulfur atom, and when Z¹ is NRa, it may or may not be linked to an A ring or a B ring to form a ring, and when Z² is NRa, it may or may not linked to a B ring or a C ring to form a ring; Ra is each independently a substituted or unsubstituted aryl group having 6 to 30 ring-forming carbon atoms, a substituted or unsubstituted heteroaryl group having 5 to 30 ring-forming carbon atoms, or a substituted or unsubstituted alkyl group having 1 to 30 carbon atoms; Y is a boron atom, a phosphorus atom, SiRb (a silicon atom having a substituent Rb), P = O or P = S; and Rb is each independently selected from a substituted or unsubstituted aryl group having 6 to 30 ring-forming carbon atoms, a substituted or unsubstituted heteroaryl group having 5 to 30 ring-forming carbon atoms, or a substituted or unsubstituted alkyl group having 1 to 30 carbon atoms.

14. A display device comprising the EL element according to any one of claims 5 to13.

15. An illumination device comprising the EL element according to any one of claims 5 to 13.

## Patentansprüche

1. Organisches EL-Elementmaterial, dargestellt durch die folgende allgemeine Formel (1): 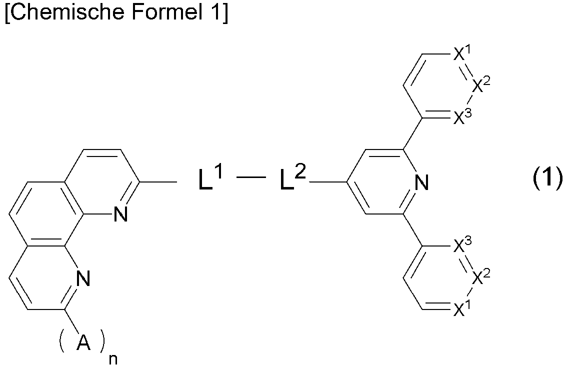 wobei in der allgemeinen Formel (1) X³ ein Stickstoffatom ist und X¹ und X² Methingruppen sind; L¹ eine substituierte oder unsubstituierte Phenylengruppe, eine substituierte oder unsubstituierte Naphthylengruppe oder eine substituierte oder unsubstituierte Anthrylengruppe ist und L² eine Einfachbindung, eine substituierte oder unsubstituierte Phenylengruppe, eine substituierte oder unsubstituierte Naphthylengruppe oder eine substituierte oder unsubstituierte Anthrylengruppe ist, wobei, wenn diese Gruppen substituiert sind, der Substituent eine Alkylgruppe oder eine Alkoxygruppe ist; A eine Phenylgruppe oder eine Pyridylgruppe ist und n 1 ist.

2. Organisches EL-Elementmaterial nach Anspruch 1, wobei in der allgemeinen Formel (1) A eine Phenylgruppe ist.

3. Organisches EL-Elementmaterial nach Anspruch 1 oder 2, wobei in der allgemeinen Formel (1) L¹ oder L² eine Naphthylengruppe ist.

4. Organisches EL-Elementmaterial nach einem der Ansprüche 1 bis 3, wobei in der allgemeinen Formel (1) L² eine Einfachbindung ist.

5. Organisches EL-Element, umfassend eine Anode und eine Kathode und mindestens eine Elektronentransportschicht und eine zwischen der Anode und der Kathode vorhandene Emissionsschicht, die ein lichtemittierendes Element ist, das Licht aus elektrischer Energie emittiert, wobei die Elektronentransportschicht das organische EL-Elementmaterial nach einem der Ansprüche 1 bis 4 enthält.

6. Organisches EL-Element nach Anspruch 5, wobei die Elektronentransportschicht ferner eine Alkalimetallkomplexverbindung enthält.

7. Organisches EL-Element, umfassend eine Anode und eine Kathode und mindestens eine Ladungserzeugungsschicht und eine zwischen der Anode und der Kathode vorhandene Emissionsschicht, die ein lichtemittierendes Element ist, das Licht aus elektrischer Energie emittiert, wobei die Ladungserzeugungsschicht das organische EL-Elementmaterial nach einem der Ansprüche 1 bis 4 enthält.

8. Organisches EL-Element nach Anspruch 7, wobei die Ladungserzeugungsschicht ferner ein Phenanthrolindimer enthält.

9. Organisches EL-Element nach Anspruch 7 oder 8, wobei die Ladungserzeugungsschicht ferner ein Alkalimetall oder ein Seltenerdmetall enthält.

10. Organisches EL-Element nach Anspruch 9, wobei das Alkalimetall Li ist.

11. Organisches EL-Element nach Anspruch 9, wobei das Seltenerdmetall Yb ist.

12. Organisches EL-Element, umfassend eine Anode und eine Kathode und mindestens eine Elektroneninjektionsschicht und eine zwischen der Anode und der Kathode vorhandene Emissionsschicht, die ein lichtemittierendes Element ist, das Licht aus elektrischer Energie emittiert, wobei die Elektroneninjektionsschicht die Verbindung nach einem der Ansprüche 1 bis 4 enthält.

13. Organisches EL-Element nach einem der Ansprüche 5 bis 12, wobei die Emissionsschicht eine Verbindung enthält, die durch die folgende allgemeine Formel (2) dargestellt ist: 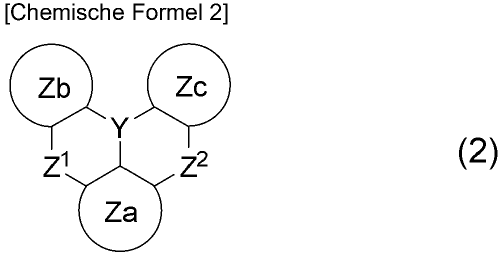 wobei in der allgemeinen Formel (2) ein Za-Ring, ein Zb-Ring und ein Zo-Ring jeweils unabhängig ein substituierter oder unsubstituierter Arylring mit 6 bis 30 ringbildenden Kohlenstoffatomen oder ein substituierter oder unsubstituierter Heteroarylring mit 5 bis 30 ringbildenden Atomen sind; Z¹ und Z² jeweils unabhängig ein Sauerstoffatom, NRa (ein Stickstoffatom mit einem Substituenten Ra) oder ein Schwefelatom sind und, wenn Z¹ NRa ist, es mit einem A-Ring oder einem B-Ring verknüpft sein kann oder nicht, um einen Ring zu bilden, und, wenn Z² NRa ist, es mit einem B-Ring oder einem C-Ring verknüpft sein kann oder nicht, um einen Ring zu bilden; Ra jeweils unabhängig eine substituierte oder unsubstituierte Arylgruppe mit 6 bis 30 ringbildenden Kohlenstoffatomen, eine substituierte oder unsubstituierte Heteroarylgruppe mit 5 bis 30 ringbildenden Kohlenstoffatomen oder eine substituierte oder unsubstituierte Alkylgruppe mit 1 bis 30 Kohlenstoffatomen ist; Y ein Boratom, ein Phosphoratom, SiRb (ein Siliciumatom mit einem Substituenten Rb), P = O oder P = S ist und Rb jeweils unabhängig aus einer substituierten oder unsubstituierten Arylgruppe mit 6 bis 30 ringbildenden Kohlenstoffatomen, einer substituierten oder unsubstituierten Heteroarylgruppe mit 5 bis 30 ringbildenden Kohlenstoffatomen oder einer substituierten oder unsubstituierten Alkylgruppe mit 1 bis 30 Kohlenstoffatomen ausgewählt ist.

14. Anzeigevorrichtung, die das EL-Element nach einem der Ansprüche 5 bis 13 umfasst.

15. Beleuchtungsvorrichtung, die das EL-Element nach einem der Ansprüche 5 bis 13 umfasst.

## Revendications

1. Matériau d'élément EL organique représenté par la formule générale (1) suivante : 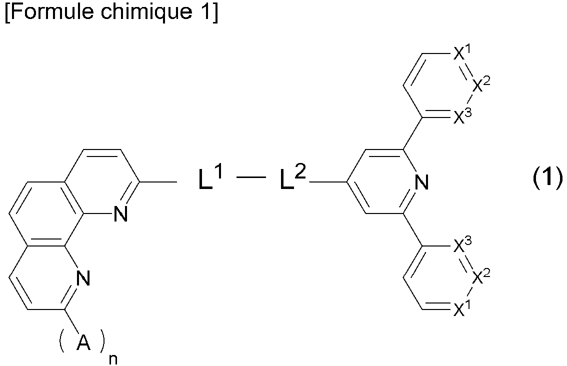 dans laquelle, dans la formule générale (1), X³ est un atome d'azote, et X¹ et X² sont des groupes méthine ; L¹ est un groupe phénylène substitué ou non substitué, un groupe naphtylène substitué ou non substitué, ou un groupe anthrylène substitué ou non substitué, et L² est une liaison simple, un groupe phénylène substitué ou non substitué, un groupe naphtylène substitué ou non substitué, ou un groupe anthrylène substitué ou non substitué, dans laquelle lorsque ces groupes sont substitués, le substituant est un groupe alkyle ou un groupe alcoxy ; A est un groupe phényle ou un groupe pyridyle ; et n vaut 1.

2. Matériau d'élément EL organique selon la revendication 1, dans lequel A est un groupe phényle dans la formule générale (1).

3. Matériau d'élément EL organique selon la revendication 1 ou 2, dans lequel L¹ ou L² est un groupe naphtylène dans la formule générale (1).

4. Matériau d'élément EL organique selon l'une quelconque des revendications 1 à 3, dans lequel L² est une liaison simple dans la formule générale (1).

5. Élément EL organique comprenant une anode et une cathode, et au moins une couche de transport d'électrons et une couche émissive existant entre l'anode et la cathode, qui est un élément électroluminescent qui émet de la lumière à partir d'énergie électrique, dans lequel la couche de transport d'électrons contient le matériau d'élément EL organique selon l'une quelconque des revendications 1 à 4.

6. Élément EL organique selon la revendication 5, dans lequel la couche de transport d'électrons contient en outre un composé complexe de métal alcalin.

7. Élément EL organique comprenant une anode et une cathode, et au moins une couche de génération de charge et une couche émissive existant entre l'anode et la cathode, qui est un élément électroluminescent qui émet de la lumière à partir d'énergie électrique, dans lequel la couche de génération de charge contient le matériau d'élément EL organique selon l'une quelconque des revendications 1 à 4.

8. Élément EL organique selon la revendication 7, dans lequel la couche de génération de charge contient en outre un dimère de phénanthroline.

9. Élément EL organique selon la revendication 7 ou 8, dans lequel la couche de génération de charge contient en outre un métal alcalin ou un métal de terres rares.

10. Élément EL organique selon la revendication 9, dans lequel le métal alcalin est Li.

11. Élément EL organique selon la revendication 9, dans lequel le métal de terres rares est Yb.

12. Élément EL organique comprenant une anode et une cathode, et au moins une couche d'injection d'électrons et une couche émissive existant entre l'anode et la cathode, qui est un élément électroluminescent qui émet de la lumière à partir d'énergie électrique, dans lequel la couche d'injection d'électrons contient le composé selon l'une quelconque des revendications 1 à 4.

13. Élément EL organique selon l'une quelconque des revendications 5 à 12, dans lequel la couche émissive contient un composé représenté par la formule générale (2) suivante : 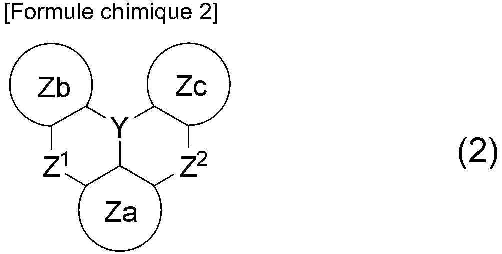 dans laquelle, dans la formule générale (2), un cycle Za, un cycle Zb et un cycle Zc sont chacun indépendamment un cycle aryle substitué ou non substitué ayant 6 à 30 atomes de carbone formant un cycle, ou un cycle hétéroaryle substitué ou non substitué ayant 5 à 30 atomes formant un cycle ; Z¹ et Z² sont chacun indépendamment un atome d'oxygène, NRa (un atome d'azote ayant un substituant Ra) ou un atome de soufre, et lorsque Z¹ est NRa, il peut ou non être lié à un cycle A ou un cycle B pour former un cycle, et lorsque Z² est NRa, il peut ou non être lié à un cycle B ou un cycle C pour former un cycle ; Ra est chacun indépendamment un groupe aryle substitué ou non substitué ayant 6 à 30 atomes de carbone formant un cycle, un groupe hétéroaryle substitué ou non substitué ayant 5 à 30 atomes de carbone formant un cycle, ou un groupe alkyle substitué ou non substitué ayant 1 à 30 atomes de carbone ; Y est un atome de bore, un atome de phosphore, SiRb (un atome de silicium ayant un substituant Rb), P = O ou P = S ; et Rb est chacun indépendamment choisi parmi un groupe aryle substitué ou non substitué ayant 6 à 30 atomes de carbone formant un cycle, un groupe hétéroaryle substitué ou non substitué ayant 5 à 30 atomes de carbone formant un cycle, ou un groupe alkyle substitué ou non substitué ayant 1 à 30 atomes de carbone.

14. Dispositif d'affichage comprenant l'élément EL selon l'une quelconque des revendications 5 à 13.

15. Dispositif d'éclairage comprenant l'élément EL selon l'une quelconque des revendications 5 à 13.
